# EUROPEAN PATENT APPLICATION

(11) **EP 3 329 996 A2**
(43) Date of publication of application: **06.06.2018**
(21) Application number: 17204026.3
(22) Date of filing: 28.11.2017
(51) Int. Cl.: B01L 7/00, G01N 35/00

(54) **SPECIMEN TREATMENT APPARATUS, SPECIMEN TREATMENT METHOD, AND SPECIMEN TREATMENT CHIP**

(30) Priority: 30.11.2016 JP 2016233705
(71) Applicant: SYSMEX CORPORATION, Kobe-shi Hyogo 651-0073 (JP)
(72) Inventor: NAKANO, Tsuyoshi, Kobe-shi, Hyogo 651-0073 (JP); YAMAKAWI, Koya, Kobe-shi, Hyogo 651-0073 (JP); TAGAWA, Ayato, Kobe-shi, Hyogo 651-0073 (JP)
(74) Representative: Hoffmann Eitle

(57) **Abstract**

Disclosed is a specimen treatment apparatus that treats a specimen by using a specimen treatment chip including a flow channel and a connecting port communicating with the flow channel, the specimen treatment apparatus including: a placement part in which the specimen treatment chip is placed; a connector provided to be engaged with the connecting port to allow a sample containing a specimen to be injected into the flow channel through the connector; and a heating unit that is pressed on the specimen treatment chip and heats the specimen treatment chip while the connector is connected to the connecting port of the specimen treatment chip placed in the placement part.

## Description

### TECHNICAL FIELD

The present invention relates to a specimen treatment apparatus, a specimen treatment method, and a specimen treatment chip.

### BACKGROUND

There is known a specimen treatment apparatus that treats a specimen by using a specimen treatment chip. For example, a flow channel including a reaction portion is formed inside a specimen treatment chip with a thickness of the order of a few millimeters. The specimen treatment apparatus sequentially moves a sample including a specimen along the flow channel inside the specimen treatment chip to cause the specimen to react under a predetermined temperature condition. In a biochemical reaction such as nucleic acid amplification, a slight difference in temperature can greatly affect a result of reaction. This requires strict temperature control for a specimen treatment chip.

For example, a structure suitable for the requirement above is described in US2005006372. As illustrated in FIG. 26, a thermal conductive connection layer 504 formed of thermal conductive grease, a thermal conductive sheet, and the like is provided between a micro chemical chip 501 and a thermal conductor 503 which is fixed to a thermoelectric element 502. A lower surface of the micro chemical chip 501 is pressed against the provided thermal conductive connection layer 504 by a movable member 505 and an elastic body 506, so as to be brought into close contact therewith. This reduces thermal resistance between the micro chemical chip 501 and the thermoelectric element 502, so that temperature of a chemical reaction portion 507 in the micro chemical chip 501 can be accurately controlled.

The configuration of US2005006372 requires a step of providing the thermal conductive connection layer 504 formed of thermal conductive grease, a thermal conductive sheet, and the like on an upper surface of the thermal conductor 503 before the micro chemical chip 501 is installed in the apparatus.

Some specimen treatment apparatuses perform treatment by sequentially injecting a sample and a reagent into a specimen treatment chip after the specimen treatment chip that is not filled with liquid is mounted in the apparatuses. In this case, a connecting port for injecting a sample and a reagent is formed in the specimen treatment chip, and each of the apparatuses is provided with a connector that is connected to the connecting port to inject the sample and the reagent. This kind of specimen treatment apparatus further requires a step of connecting the connecting port in the chip and the connector in the apparatus to each other after the specimen treatment chip is placed.

As described above, a specimen treatment apparatus using a specimen treatment chip requires various steps when the specimen treatment chip is mounted. It is preferable to simplify these steps as much as possible to improve operability.

In light of the problems above, it is an object of the present invention to provide a specimen treatment apparatus, a specimen treatment method, and a specimen treatment chip that are capable of accurately controlling temperature of a specimen treatment chip, as well as of improving operability when the specimen treatment chip is mounted.

### SUMMARY OF THE INVENTION

A first aspect of the present invention relates to a specimen treatment apparatus (10 or 100) that treats a specimen by using a specimen treatment chip (20 or 200) including a flow channel (21, or each of 210 to 260) and a connecting port (22, or each of 271 to 281) communicating with the flow channel (21, or each of 210 to 260). The specimen treatment apparatus (10 or 100) according to the present aspect includes: a placement part (30 or 160) in which the specimen treatment chip (20 or 200) is placed; a connector (50 or 140) provided to be engaged with the connecting port (22, or each of 271 to 281) to allow a sample containing a specimen to be injected into the flow channel (21, or each of 210 to 260) through the connector; and a heating unit (40, 170a, or 170b) that is pressed on the specimen treatment chip (20 or 200) and heats the specimen treatment chip (20 or 200) while the connector (50 or 140) is connected to the connecting port (22, or each of 271 to 281) of the specimen treatment chip (20 or 200) placed in the placement part (30 or 160).

In the present aspect, the specimen treatment chip is an exchangeable component in which a flow channel is formed in a pattern required for specimen treatment. In the specimen treatment chip, a reagent may be preliminarily contained in a predetermined part of the flow channel, or a reagent may be injected from the connector through a predetermined connecting port. The sample can contain a reagent, a cleaning liquid, a dilution, and the like, for a reaction. The connector may be directly connected to the connecting port, or the connector may be indirectly connected to the connecting port. When the connector is indirectly connected to the connecting port, the connector is connected, for example, to a reservoir for storing a reagent or the like, provided in the connecting port. Then, the connector applies pressure to the reservoir so that a sample or the like is injected into the flow channel from the reservoir through the connecting port.

In the specimen treatment apparatus according to the present aspect, the heating unit pressed on the specimen treatment chip transmits heat of the heating unit to the specimen treatment chip while the connecting port of the specimen treatment chip placed in the placement part is connected to the connector. At this time, the heating unit is brought into close contact with an outer surface of the specimen treatment chip to eliminate a gap between the specimen treatment chip and the heating unit. This allows heat of the heating unit to be smoothly transmitted to the specimen treatment chip, so that temperature of the specimen treatment chip can be accurately controlled. Connecting the connector to the connecting port, and heating the specimen treatment chip with the heating unit, are simultaneously performed. This enables a step of mounting a specimen treatment chip to be simplified, so that operability when the specimen treatment chip is mounted can be improved.

In the specimen treatment apparatus (10 or 100) according to the present aspect, the heating unit (40, 170a, or 170b) can be configured so as to include a heat generator (41, 171, or 173) that generates heat, and an elastic member (42, 172, or 174) provided on the heat generator (41, 171, or 173).

In the specimen treatment apparatus (10 or 100) according to the present aspect, the heating unit (40) can be configured so as to include a heat generator (41) that generates heat, and an elastic member (42) that is formed integrally with the heat generator (41).

The specimen treatment apparatus (10 or 100) according to the present aspect can be configured so as to include a moving member (60 or 130) that can connect the connector (50 or 140) to the connecting port (22, or each of 271 to 281) and can press the heating unit (40, 170a, or 170b) on the specimen treatment chip (20 or 200). This enables connection of the connector to the connecting port and pressing of the heating unit on the specimen treatment chip to be simultaneously performed by only operating the moving member.

In the specimen treatment apparatus (10 or 100) according to the present aspect, the moving member (60 or 130) can be configured so as to move the connector (50 or 140) to connect the connector (50 or 140) to the connecting port (22, or each of 271 to 281), and to press the heating unit (40, 170a, or 170b) on the specimen treatment chip (20 or 200).

In the specimen treatment apparatus (10 or 100) according to the present aspect, the connector (50 or 140) can be provided in the moving member (60 or 130).

The specimen treatment apparatus (100) according to the present aspect can be configured to include a body (110), and a lid part (120) that is supported by the body (110) to be openable and closable. In this case, the lid part (120) can be configured so as to be closed to connect the connector (140) to the connecting port (each of 271 to 281), and to press the heating unit (170a or 170b) on the specimen treatment chip (200). This enables the connector to be connected to the connecting port and the heating unit to press the specimen treatment chip by only closing the lid part.

In this case, the connector (140) can be provided in the lid part (120). This enables the specimen treatment apparatus to be simply configured because no mechanism is required to be separately provided to connect the connector to the connecting port.

In the specimen treatment apparatus (10 or 100) according to the present aspect, the specimen treatment chip (20 or 200) can be configured to include a plurality of connecting ports (22, or 271 to 281). In this case, the specimen treatment apparatus (10 or 100) according to the present aspect can be configured to include a plurality of connectors (50 or 140) provided to be engaged with the plurality of connecting ports (22, or 271 to 281), respectively, to inject a reagent for treating a specimen into the flow channel through the plurality of connecting ports (22, or 271 to 281). Even when a plurality of connectors is provided as described above, the plurality of connectors can be smoothly connected to the corresponding connecting ports.

In this case, the heating unit (40, 170a, or 170b) may be disposed on an opposite side to the plurality of connectors (50 or 140) with respect to the specimen treatment chip (20 or 200) placed in the placement part (30 or 160).

The heating unit (40, 170a, or 170b) can be disposed between a first connector (50 or 140) and a second connector (50 or 140) of the plurality of connectors (50 or 140).

The specimen treatment apparatus (10 or 100) according to the present aspect can be configured so as to include a locking part (122) that locks a member (60, 120, or 130) for connecting the connector (50 or 140) to the connecting port (22, or each of 271 to 281) and pressing the elastic member (42, 172, or 174) on the specimen treatment chip (20 or 200). Heat of the heat generator is transmitted to the specimen treatment chip through the elastic member. Therefore, the heat generator is not required to press the specimen treatment chip with a strong force, which is required when no elastic member is used. Thus, after the connector is connected to the connecting port to press the elastic member on the specimen treatment chip, the moving member may only be locked to the extent that the moving member does not move. This enables the specimen treatment apparatus to be simply configured because a configuration for continuously applying a strong force is unnecessary.

In the specimen treatment apparatus (10 or 100) according to the present aspect, the heating unit (40, 170a, or 170b) and the connector (50 or 140) may be provided in the member (60, 120, or 130) for connecting the connector (50 or 140) to the connecting port (22, or each of 271 to 281) and pressing the elastic member (42, 172, or 174) on the specimen treatment chip (20 or 200), the heat generator (41, 171, or 173) may be disposed on the elastic member (42, 172, or 174), and the connector (50 or 140) may be disposed to be lateral to the heating unit (40, 170a, or 170b). This allows another member for treating a specimen, such as a magnet, to be easily disposed in the body of the specimen treatment apparatus.

In this case, the member (60, 120, or 130) can be configured to include a plane (61 or 131) defining a bonding surface of the connector (50 or 140), and a recessed portion (62 or 132) recessed from the plane (61 or 131) and having a heating unit (40, 170a, or 170b) being disposed therein. The recessed portion (62 or 132) has a height larger than a thickness of the heat generator (41, 171, or 173), and less than a sum of thicknesses of the heat generator (41, 171, or 173) and the elastic member (42, 172, or 174). This enables the elastic member to be deformed to be brought into close contact with the specimen treatment chip when the connector is connected to the connecting port by the moving member.

In this case, the recessed portion (62 or 132) can be configured to have an inner dimension larger than the elastic member (42, 172, or 174). This causes a clearance between the elastic member and the recessed portion, so that the deformed elastic member is prevented from protruding from the plane defining the bonding surface of the connector. Thus, the connector and the connecting port can be connected to each other without a clearance.

A difference between a height of the recessed portion (62 or 132), and a sum of thicknesses of the heat generator (41, 171, or 173) and the elastic member (42, 172, or 174), can be configured to be 1.7 mm or less. A force applied to the specimen treatment chip is a total of a force pressing the elastic member and a force allowing the connector to be connected to the connecting port. Thus, when the difference is set to be low as described above, a force to be applied to the specimen treatment chip can be reduced. As a result, it is possible to prevent deformation of the specimen treatment chip and clogging of the flow channel caused by deformation of the specimen treatment chip. The above difference is not limited to be set to 1.7 mm or less. The above difference may be set depending on a height of the recessed portion, a thickness of the heat generator, and a thickness of the elastic member.

The specimen treatment apparatus (10 or 100) according to the present aspect can be configured such that the elastic member (42, 172, or 174) has a thickness of 0.3 mm or more and 2.0 mm or less. This enables the elastic member to be deformed to the extent that a clearance between the heat generator and the specimen treatment chip can be eliminated. Then, heat generated by the heat generator can be smoothly transmitted to the specimen treatment chip.

The specimen treatment apparatus (10 or 100) according to the present aspect can be configured such that a plurality of heating units (170a and 170b) is disposed in association with regions (213a, 213b, and 213c, or 232a, 232b, and 232c) different from each other of the flow channel (210 or 230). This enables different regions of the flow channel to be heated at temperatures different from each other. As a result, temperature control can be finely performed.

The specimen treatment apparatus (10 or 100) according to the present aspect can be configured such that the specimen treatment chip (20 or 200) is used for nucleic acid amplification, the plurality of heating units (40, or 170a and 170b) is disposed in association with respective flow channel portions (213a, 213b, and 213c, or 232a, 232b, and 232c) for corresponding processes of amplification, extension, and bonding of nucleic acid, and each of the plurality of heating units (170a and 170b) is heated to the corresponding one of temperatures appropriate for the respective processes of amplification, extension, and bonding of nucleic acid. As a result, temperatures appropriate for respective processes of amplification, extension, and bonding of nucleic acid can be applied to the corresponding flow channel portions, so that temperature control during the nucleic acid amplification can be performed with high accuracy and reliably.

The specimen treatment apparatus (10 or 100) according to the present aspect can be configured such that the flow channel (210 or 230) meanders with a predetermined amplitude, each of the plurality of heating units (170a and 170b) is disposed in the corresponding one of a first region (213a or 232a) on one of sides across a middle portion of the meander, a second region (213b or 232b) at the middle portion of the meander, and a third region (213c or 232c) on the other of sides across the middle portion of the meander, and the heating units (170a and 170b) disposed in the corresponding first region (213a or 232a), second region (213b or 232b), and third region (213c or 232c), are controlled to be respective temperatures appropriate for the respective processes of amplification, extension, and bonding of nucleic acid. As a result, three temperatures can be applied to a sample by only allowing the sample to flow through the flow channel meandering. This enables thermal cycle treatment during the nucleic acid amplification to be easily performed, for example.

The specimen treatment apparatus (10 or 100) according to the present aspect includes a reagent containing section (153) that contains a reagent for the nucleic acid amplification. In this case, a plurality of connecting ports (271 to 281) can be provided in the specimen treatment chip (200), a plurality of connectors (140) can be provided to be engaged with the respective connecting ports (271 to 281), and the reagent sucked from the reagent containing section (153) can be injected into the flow channels (210 to 260) of the specimen treatment chip (200) through the corresponding connectors (140). As a result, the nucleic acid amplification can be smoothly performed by using the specimen treatment chip.

The specimen treatment apparatus (10 or 100) according to the present aspect can be configured such that pressure to be applied to the specimen treatment chip (20 or 200) is 85.6 kPa or more and 839.5 kPa or less while the connector (50 or 140) is connected to the connecting port (22, or each of 271 to 281). When pressure applied to the specimen treatment chip is more than the above pressure, the specimen treatment chip is liable to deform due to heat generated by the heating unit. In this case, the flow channel of the specimen treatment chip is narrowed, so that the sample and the reagent are less likely to flow into the flow channel. In contrast, when pressure to be applied to the specimen treatment chip is set as described above, deformation of the specimen treatment chip is reduced. As a result, the sample and the reagent can be prevented from being less likely to flow into the flow channel.

In the specimen treatment apparatus (10 or 100) according to the present aspect, the connector (50 or 140) can be configured to be connected to the connecting port (22, or each of 271 to 281) by being pressed on the connecting port (22, or each of 271 to 281).

The specimen treatment apparatus (10 or 100) according to the present aspect can be configured so as to include a support member (33) that is disposed on an opposite side to the connector (50 or 140) with respect to the specimen treatment chip (20 or 200) placed in the placement part (30 or 160) to support the specimen treatment chip (20 or 200). As a result, the connector and the connecting port can be reliably connected to each other. Then, a force applied to the specimen treatment chip from the connector can prevent the specimen treatment chip from bending.

In this case, the specimen treatment apparatus (10 or 100) according to the present aspect can be configured so as to include a cushioning member (34) provided in the support member (33), and the support member (33) can be configured so as to support the specimen treatment chip (20 or 200) with the cushioning member (34). As a result, a force to be applied to the specimen treatment chip from the support member can be distributed, so that deformation of the specimen treatment chip can be reduced as compared with when the support member directly supports the specimen treatment chip to enable breakage of the specimen treatment chip to be prevented. The cushioning member is formed of rubber, for example.

In the specimen treatment apparatus (10 or 100) according to the present aspect, the elastic member (42, 172, or 174) can be formed of thermal conductive silicone rubber. As a result, heat generated by the heat generator can be efficiently transmitted to the specimen treatment chip.

In the specimen treatment apparatus (10 or 100) according to the present aspect, the heat generator (41, 171, or 173) can be composed of a heater block. As a result, a predetermined area on the specimen treatment chip can be smoothly heated.

The specimen treatment apparatus (10 or 100) according to the present aspect can be configured such that the heating unit (40, 170a, or 170b) is disposed above the specimen treatment chip (20 or 200) placed in the placement part (30 or 160). The specimen treatment apparatus (10 or 100) according to the present aspect also can be configured so as to include an elastic member (35) that is provided in a supporting surface (31a) of the placement part (30 or 160), supporting the specimen treatment chip (20 or 200). As a result, a force to be applied to the specimen treatment chip from the supporting surface can be distributed, so that deformation of the specimen treatment chip can be reduced as compared with when the supporting surface directly supports the specimen treatment chip to enable breakage of the specimen treatment chip to be prevented. In this case, the elastic member is formed of rubber, for example.

A second aspect of the present invention relates to a specimen treatment method for treating a specimen by using a specimen treatment chip (20 or 200) including a flow channel (21, or each of 210 to 260) and a connecting port (22, or each of 271 to 281) communicating with the flow channel (21, or each of 210 to 260). The specimen treatment method according to the present aspect includes the steps of: connecting the connector (50 or 140) to the connecting port (22, or each of 271 to 281), the connector allowing a sample containing a specimen to be injected into the flow channel (21, or each of 210 to 260) through the connector; and pressing the heating unit (40, 170a, or 170b) that heats the specimen treatment chip (20 or 200), on the specimen treatment chip (20 or 200).

The specimen treatment method according to the present aspect achieves an effect similar to that of the first aspect.

In the specimen treatment method according to the present aspect, a specimen or a reagent for treating the specimen is injected into the flow channel (21, or each of 210 to 260) from the connector (50 or 140) through the connecting port (22, or each of 271 to 281).

In the specimen treatment method according to the present aspect, the specimen treatment chip (20 or 200) is heated with the heating unit (40, 170a, or 170b) pressed on the specimen treatment chip (20 or 200).

In the specimen treatment method according to the present aspect, the connector (50 or 140) is moved to connect the connector (50 or 140) to the connecting port (22, or each of 271 to 281), and to press the heating unit (40, 170a, or 170b) on the specimen treatment chip (20 or 200).

In the specimen treatment method according to the present aspect, a lid part (120) is closed to connect the connector (140) to the connecting port (each of 271 to 281), and to press the heating unit (170a or 170b) on the specimen treatment chip (20 or 200).

In the specimen treatment method according to the present aspect, the connector (50 or 140) is connected to the connecting port (22, or each of 271 to 281) and the heating unit (40, 170a, or 170b) is pressed on the specimen treatment chip (20 or 200), by using a pressure of 85.6 kPa or more and 839.5 kPa or less.

In the specimen treatment method according to the present aspect, the specimen treatment chip (20 or 200) is used for nucleic acid amplification, and heat appropriate for respective processes of amplification, extension, and bonding of nucleic acid is applied to the corresponding flow channel portions (213a, 213b, and 213c, or 232a, 232b, and 232c) for the corresponding processes of amplification, extension, and bonding of nucleic acid.

A third aspect of the present invention relates to a specimen treatment method for treating a specimen by using a specimen treatment chip (20 or 200) including a flow channel (21, or each of 210 to 260) and a connecting port (22, or each of 271 to 281) communicating with the flow channel (21, or each of 210 to 260). In the specimen treatment method according to the present aspect, a heating unit (40, 170a, or 170b) heats the specimen treatment chip (20 or 200) while a connector (50 or 140) is connected to the connecting port (22, or each of 271 to 281) and the heating unit (40, 170a, or 170b) is pressed on the specimen treatment chip (20 or 200).

The specimen treatment method according to the present aspect achieves an effect similar to that of the first aspect.

In the specimen treatment method according to the present aspect, a specimen or a reagent for treating the specimen is injected into the flow channel (21, or each of 210 to 260) from the connector (50 or 140) through the connecting port (22, or each of 271 to 281).

In the specimen treatment method according to the present aspect, a lid part (120) is closed to connect the connector (140) to the connecting port (each of 271 to 281), and to press the heating unit (170a or 170b) on the specimen treatment chip (20 or 200).

In the specimen treatment method according to the present aspect, the connector (50 or 140) is connected to the connecting port (22, or each of 271 to 281) and the heating unit (40, 170a, or 170b) is pressed on the specimen treatment chip (20 or 200), by using a pressure of 85.6 kPa or more and 839.5 kPa or less.

A fourth aspect of the present invention relates to a specimen treatment chip (20). The specimen treatment chip (20) according to the present aspect includes a flow channel (21), a connecting port (22) communicating with the flow channel (21), and an elastic member (23) fixed to a region (24a, 24b, or 24c) corresponding to a portion of the flow channel (21) where temperature control is needed.

In the specimen treatment chip according to the present aspect, the elastic member is provided in the specimen treatment chip, so that the elastic member does not need to be provided in a specimen treatment apparatus. The elastic member is provided in the specimen treatment chip, so that a fresh elastic member is to be used for each processing of a specimen. When a fresh elastic member is used every time as described above, a deteriorated elastic member can be prevented from being used. Thus, when a heat generator that generates heat is pressed on the elastic member, temperature control using the heat generator can be reliably performed.

In the specimen treatment chip (20) according to the present aspect, the flow channel (21) meanders with a predetermined amplitude, and the elastic member (23) is individually fixed to a first region (24a) on one of sides across a middle portion of the meander, a second region (24b) at the middle portion of the meander, and a third region (24c) on the other of sides across the middle portion of the meander. As a result, when the heat generators, each of which generates different heat, are pressed on the corresponding elastic members associated with the respective first to third regions, for example, three temperatures can be applied to a sample by only allowing the sample to flow through the flow channel meandering. This enables thermal cycle treatment during the nucleic acid amplification to be easily performed, for example.

According to the present invention, temperature control for a specimen treatment chip can be accurately performed, and operability at the time of mounting a specimen treatment chip can be improved.

### BRIEF DESCRIPTION OF THE DRAWINGS

Figs. 1A and 1B are schematic diagrams illustrating a structure of a specimen treatment apparatus according to an embodiment 1.
Fig. 2 is a flowchart to describe an example of performing an emulsion PCR assay.
Figs. 3A to 3G are schematic diagrams illustrating a progression of an emulsion PCR assay.
Fig. 4 is a schematic diagram illustrating an appearance of a specific structure of the specimen treatment apparatus according to the embodiment 1.
Figs. 5A and 5B are schematic diagrams illustrating a specific structure of a locking part according to the embodiment 1.
Fig. 6 is a schematic diagram illustrating an appearance of a modification of the specific structure of the specimen treatment apparatus according to the embodiment 1.
Fig. 7A is a schematic diagram of a specific structure of a specimen treatment chip according to the embodiment 1 as viewed from above.
Fig. 7B is a schematic diagram of a specific structure of a moving member and a connector, according to the embodiment 1, as viewed from below.
Fig. 8 is a block diagram illustrating a specific configuration of a liquid feeder according to the embodiment 1.
Fig. 9A is a schematic diagram illustrating a placement part, a heating unit, a magnet, and a heat generator, in a specific structure according to the embodiment 1, as viewed from above.
Fig. 9B is a schematic diagram illustrating the placement part, the heating unit, the magnet, and the heat generator, in the specific structure, according to the embodiment 1, as viewed from a Y-axis positive direction.
Fig. 9C is a schematic diagram illustrating a state of a specific structure according to the embodiment 1, where an elastic member is pressed on a specimen treatment chip and a connector is connected to a connecting port.
Fig. 10A is a schematic diagram illustrating a flow channel of a specific structure according to the embodiment 1, in which Pre-PCR treatment is performed.
Fig. 10B is a schematic diagram illustrating a flow channel of a specific structure according to the embodiment 1, in which emulsion forming treatment is performed.
Fig. 10C is a schematic diagram to illustrate forming of an emulsion in a specific structure according to the embodiment 1.
Fig. 11A is a schematic diagram illustrating a flow channel of a specific structure according to the embodiment 1, in which emulsion PCR treatment is performed.
Fig. 11B is a schematic diagram illustrating a flow channel of a specific structure according to the embodiment 1, in which emulsion breaking treatment is performed.
Fig. 12A is a schematic diagram illustrating a structure of a flow channel of a specific structure according to the embodiment 1, in which primary cleaning and denaturation treatment are performed.
Fig. 12B is a schematic diagram illustrating a flow channel of a specific structure according to the embodiment 1, in which hybridization treatment is performed.
Fig. 13 is a block diagram illustrating a specific configuration of a specimen treatment apparatus according to the embodiment 1.
Figs. 14A to 14C are photographs respectively showing temperature distributions of specimen treatment chips of verification examples 1 to 3, acquired per one measurement.
Figs. 14D to 14F are illustrations schematically showing states of Figs. 14A to 14C, respectively.
Figs. 15A to 15C are graphs showing temperature variations acquired in the verification examples 1 to 3 in respective regions.
Figs. 16A and 16B are schematic diagrams illustrating a structure of a specimen treatment apparatus according to an embodiment 2.
Figs. 17A and 17B are schematic diagrams illustrating a structure of a specimen treatment apparatus according to an embodiment 3.
Fig. 18A is a schematic diagram of the vicinity of an elastic member according to the embodiment 3 as viewed from a lower surface side of a moving member.
Figs. 18B and 18C are schematic diagrams illustrating a height of a recessed portion, a thickness of a heat generator, a thickness of an elastic member, and a clearance between the recessed portion and the elastic member, according to the embodiment 3.
Fig. 19 is a schematic diagram illustrating an appearance of a specific structure of the specimen treatment apparatus according to the embodiment 3.
Fig. 20 is a schematic diagram illustrating an appearance of a specific structure of a specimen treatment apparatus according to an embodiment 4.
Fig. 21 is a schematic diagram illustrating an appearance of a specific structure of the specimen treatment apparatus according to the embodiment 4.
Figs. 22A and 22B are schematic diagrams illustrating a structure of a specimen treatment apparatus according to an embodiment 5.
Figs. 23A and 23B are schematic diagrams illustrating a structure of a specimen treatment apparatus according to an embodiment 6.
Figs. 24A and 24B are schematic diagrams illustrating a structure of a specimen treatment apparatus according to an embodiment 7.
Fig. 25A is a schematic diagram illustrating a structure of a specimen treatment chip according to an embodiment 8.
Fig. 25B is a schematic diagram of the specimen treatment chip according to the embodiment 8 as viewed from a lower surface side of the specimen treatment chip.

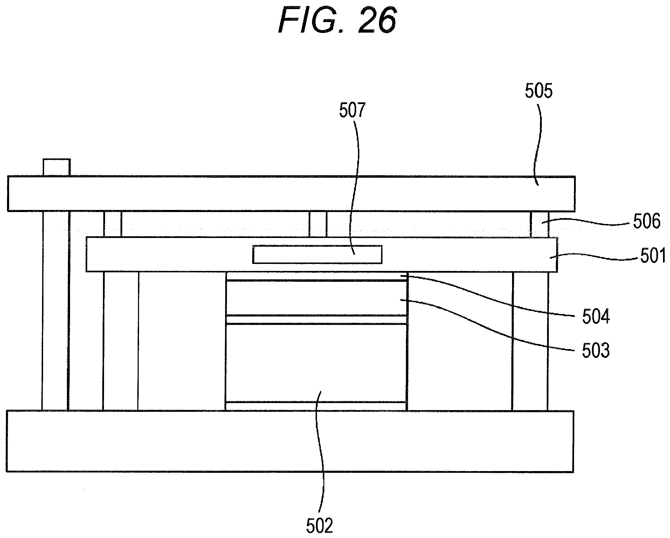

Fig. 26 is a schematic diagram to illustrate a structure according to related art.

### DETAILED DESCRIPTION OF THE PREFERRED EMBODIMENTS

### <Embodiment 1>

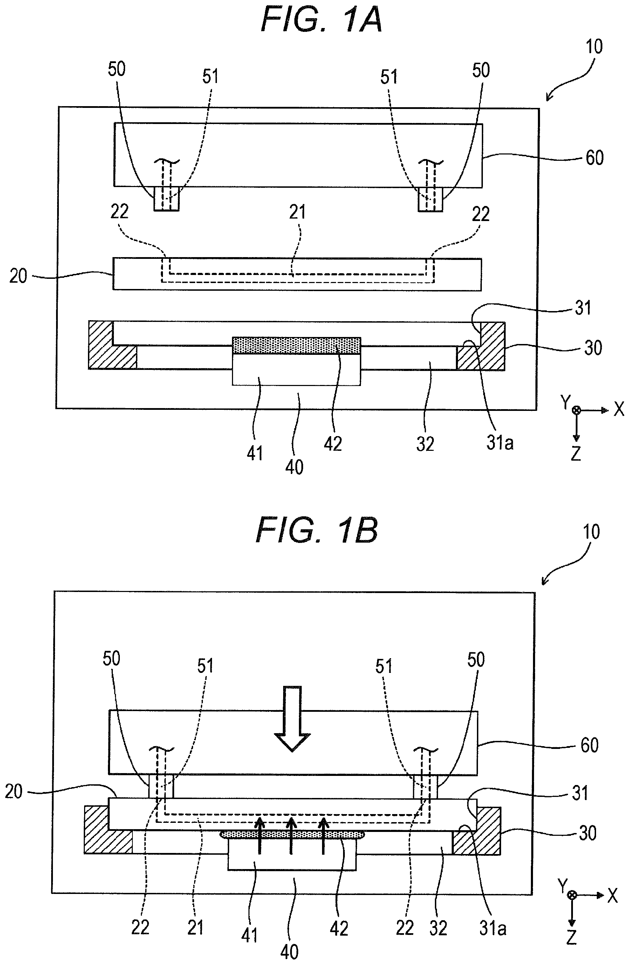

With reference to Figs. 1A and 1B, a specimen treatment apparatus of an embodiment 1 will be described.

As illustrated in Fig. 1A, a specimen treatment apparatus 10 treats a specimen using a specimen treatment chip 20. The specimen treatment apparatus 10 includes a placement part 30, a heating unit 40, a connector 50, and a moving member 60. In Fig. 1A, XYZ-axes are orthogonal to each other. An X-axis positive direction indicates a right direction, a Y-axis positive direction indicates a rear direction, and a Z-axis positive direction indicates a vertically downward direction. An XY-plane is parallel to a horizontal plane. Fig. 1A is an illustration of the specimen treatment apparatus 10 as viewed backward from its front face.

The specimen treatment chip 20 is an exchangeable component including functions required for treatment of a specimen. The specimen treatment chip 20 has the shape of a flat plate. The specimen treatment chip 20 includes a flow channel 21, and a connecting port 22 communicating with the flow channel 21. The connecting port 22 is formed of an outlet of a hole provided in an upper surface of the specimen treatment chip 20. The specimen treatment chip 20 illustrated in Fig. 1A as an example includes two connecting ports 22, and the two connecting ports 22 communicate with each other through the flow channel 21. A flat-shaped substrate constituting the specimen treatment chip 20 is a rigid member, such as that formed of a cycloolefin polymer or glass. The specimen treatment chip 20 has a thickness of 0.3 mm or more and 5 mm or less. The flow channel 21 has sectional dimensions, or a width, a height, an inner dimension, and the like, each of which is 0.1 µm or more and 1000 µm or less.

In the placement part 30, the specimen treatment chip 20 is placed. Fig. 1A illustrates a cut surface of the placement part 30, taken along an XZ-plane, as viewed from the Y-axis positive direction. The placement part 30 is provided with a recessed portion 31. In the horizontal plane, the recessed portion 31 has a size that is substantially identical to that of the specimen treatment chip 20. The recessed portion 31 has a bottom surface 31 a that is provided at its central portion with a hole 32. The hole 32 vertically passes through the placement part 30. The bottom surface 31 a is a supporting surface that supports the specimen treatment chip 20 upward. When the specimen treatment chip 20 is placed in the placement part 30, a position of the specimen treatment chip 20 in the horizontal plane is defined by side surfaces of the recessed portion 31.

The heating unit 40 is provided inside the specimen treatment apparatus 10. The heating unit 40 heats the specimen treatment chip 20 placed in the placement part 30. The heating unit 40 includes a heat generator 41 and an elastic member 42.

The heat generator 41 is formed by providing a flat member with high thermal conductivity on a heat source. The heat source of the heat generator 41 is formed by winding around a rectangular column, for example. An outer surface of the flat member of the heat generator 41 constitutes a heat generating surface of the heat generator 41. When an electric current is applied to the heat source of the heat generator 41, the heat source generates heat, and the generated heat is transmitted to the heat generating surface of the heat generator 41. In Fig. 1A, the heat generating surface of the heat generator 41 is an upper surface of the heat generator 41. The heat generator 41 is composed of a heater block, for example. This enables a predetermined area on the specimen treatment chip 20 to be smoothly heated.

The elastic member 42 transmits heat generated by the heat generator 41 to the specimen treatment chip 20. The elastic member 42 is provided in the upper surface of the heat generator 41. The elastic member 42 is bonded to the upper surface of the heat generator 41 with elastic silicone rubber-based adhesive, for example. The elastic member 42 may be provided on the upper surface of the heat generator 41 by using adhesiveness of the elastic member 42 itself. When the elastic member 42 is provided on the upper surface of the heat generator 41, an operator does not need to preliminarily interpose the elastic member 42 between the specimen treatment chip 20 and the heat generator 41 when treating a specimen by using the specimen treatment apparatus 10 and the specimen treatment chip 20. This enables reduction in complicatedness of operation by an operator.

The elastic member 42 is formed of thermal conductivity silicone rubber, for example. This enables heat generated by the heat generator 41 to be efficiently transmitted to the specimen treatment chip 20. As a material constituting the elastic member 42, silicone resin or acrylic-based elastomer is suitable, for example. Specifically, as a material constituting the elastic member 42, a silicone rubber sheet (BA grade) made by Shin-Etsu Chemical Co., Ltd is available. It is desirable that a material constituting the elastic member 42 has high thermal conductivity. For example, when a material acquired by mixing filler such as silica and alumina in resin is used as a material constituting the elastic member 42, thermal conductivity of the elastic member 42 can be increased. Specifically, as a material constituting the elastic member 42, thermal conductivity silicone rubber with a thermal conductivity of about 1.3 W/m·K, made by Shin-Etsu Chemical Co., Ltd, is available. Besides this, a material constituting the elastic member 42 is not particularly limited as long as the material is elastically deformed by pressing force.

While a thickness of the elastic member 42 is not particularly limited, the elastic member 42 has an extremely small amount of elastic deformation when being too thin. In this case, a gap existing between a lower surface of the specimen treatment chip 20 and the upper surface of the heat generator 41 cannot be eliminated, so that accuracy of temperature control for the specimen treatment chip 20 cannot be improved. Meanwhile, too much thickness of the elastic member 42 is undesirable because heat transmission from the heat generator 41 to the specimen treatment chip 20 requires time. Thus, the elastic member 42 may have a thickness of 0.3 mm or more and 2.0 mm or less, preferably has a thickness of 0.5 mm or more and 1.5 mm or less, and most preferably has a thickness of 0.8 mm or more and 1.2 mm or less.

When a thickness of the elastic member 42 is set as described above, the elastic member 42 can be deformed to the extent that a clearance between the specimen treatment chip 20 and the heat generator 41 can be eliminated. Then, heat generated by the heat generator 41 can be smoothly transmitted to the specimen treatment chip 20.

The elastic member 42 may have a shape in the horizontal plane, the shape corresponding to a region required for temperature control for the specimen treatment chip 20. For example, the elastic member 42 may have a shape identical to that of the upper surface of the heat generator 41 in the horizontal plane, as illustrated in Fig. 1A. In the horizontal plane, the elastic member 42 may also have a shape identical to that of the specimen treatment chip 20.

The upper surface of the heat generator 41 is positioned below the bottom surface 31 a of the recessed portion 31. An upper surface of the elastic member 42 provided on the upper surface of the heat generator 41 is positioned above the bottom surface 31 a of the recessed portion 31. As a result, when the specimen treatment chip 20 is fitted into the recessed portion 31 from above, the lower surface of the specimen treatment chip 20 is brought into contact with the upper surface of the elastic member 42 before being brought into contact with the bottom surface 31 a of the recessed portion 31.

The connector 50 is provided in a lower surface of the moving member 60. In the example illustrated in Fig. 1A, the two connectors 50 are provided in the lower surface of the moving member 60 while being spaced in a side-to-side direction. In the embodiment 1, the heating unit 40 is disposed on an opposite side to the connectors with respect to the specimen treatment chip 20 placed in the placement part 30. The connector 50 has a lower surface that is a plane parallel to the horizontal plane. The connector 50 is high in a vertical direction, and is in the shape of a rectangular parallelepiped or a circular cylinder. The connector 50 is provided with a hole 51 thereinside. The hole 51 passes through the connector 50 in the vertical direction. The connector 50 is provided to be engaged with the connecting port 22 of the specimen treatment chip 20 placed in the placement part 30. The connector 50 is used to inject a reagent for treating a sample containing a specimen and the specimen into the flow channel 21 of the specimen treatment chip 20.

The connector 50 may be provided in the lower surface of the moving member 60 in accordance with the number of the connecting ports 22 provided in the specimen treatment chip 20, and a procedure for treating the specimen in the specimen treatment chip 20. That is, the number of the connectors 50 to be provided in the moving member 60 is not limited to two, and may be one, or three or more, in accordance with the number of the connecting ports 22, and the procedure for treating the specimen. When a plurality of connectors 50 is provided, the heating unit 40 is disposed between one of the plurality of connectors 50 and another connector 50.

When a specimen is treated, first the specimen treatment chip 20 is placed in the recessed portion 31. Subsequently, the moving member 60 is moved downward from the position illustrated in Fig. 1A to move the connector 50 downward. As a result, the upper surface of the specimen treatment chip 20 is pressed downward by the lower surface of the connector 50 as illustrated in Fig. 1B, so that the lower surface of the specimen treatment chip 20 is pressed on the bottom surface 31 a of the recessed portion 31.

As illustrated in Fig. 1B, when the connector 50 is moved downward until the lower surface of the specimen treatment chip 20 is brought into contact with the bottom surface 31 a of the recessed portion 31, the connector 50 is connected to the connecting port 22 and the lower surface of the specimen treatment chip 20 is pressed on the elastic member 42. As described above, the moving member 60 moves the plurality of connectors 50 downward to connect the plurality of connectors 50 to the corresponding connecting ports 22, and presses the heating unit 40 on the specimen treatment chip 20. According to the embodiment 1, even when a plurality of connectors 50 is provided, the moving member 60 moves the plurality of connectors 50 to enable the plurality of connectors 50 to be smoothly connected to the corresponding connecting ports 22.

A force pressing the lower surface of the connector 50 on the specimen treatment chip 20 may be necessary to the extent that the connector 50 can be connected to the connecting port 22 and the elastic member 42 can be elastically deformed to eliminate a gap between the specimen treatment chip 20 and the heat generator 41.

Pressure to be applied to the specimen treatment chip 20 is set to 85.6 kPa or more and 839.5 kPa or less while the connector 50 is connected to the connecting port 22 by the moving member 60. The pressure to be applied to the specimen treatment chip 20 is determined on the basis of a load from the connector 50 and a load from the elastic member 42. When the pressure applied to the specimen treatment chip 20 by the moving member 60 is more than the above pressure, the specimen treatment chip 20 is liable to deform due to heat generated by the heating unit 40. In this case, the flow channel 20 of the specimen treatment chip is narrowed, so that the sample and the reagent do not smoothly flow into the flow channel 21. In contrast, when pressure to be applied to the specimen treatment chip 20 by the moving member 60 is set as described above, deformation of the specimen treatment chip 20 is reduced. As a result, the sample and the reagent can be prevented from being less likely to flow into the flow channel 21.

When a force applied to the specimen treatment chip 20 concentrates at a small area, the specimen treatment chip 20 may be deformed. Thus, it is desirable that a force applied to the specimen treatment chip 20 is distributed into a large area as much as possible, as long as the connector 50 is connected to the connecting port 22 and a gap between the specimen treatment chip 20 and the heat generator 41 is eliminated. To distribute a force applied to the specimen treatment chip 20 into a large area, a member for pressing the upper surface of the specimen treatment chip 20 on the lower surface of the moving member 60 may be provided, for example.

Subsequently, a sample containing a specimen and a reagent for treating the specimen are injected into the specimen treatment chip 20 through the hole 51 of the connector 50 and the connecting port 22 of the specimen treatment chip 20. This allows the sample and the reagent to be injected into the flow channel 21 of the specimen treatment chip 20. Then, the heat generator 41 is driven. As indicated by bold solid line arrows in Fig. 1B, heat generated by the heat generator 41 is transmitted to the specimen treatment chip 20 through the elastic member 42. This allows the sample passing through the flow channel 21 to be heated to promote reaction in the sample.

According to the embodiment 1, when the connector 50 is moved by the moving member 60 after the specimen treatment chip 20 is placed in the placement part 30, the connector 50 is connected to the connecting port 22 and the heating unit 40, specifically the elastic member 42, is pressed on the specimen treatment chip 20. As a result, the elastic member 42 interposed between the specimen treatment chip 20 and the heat generator 41 is deformed, so that the elastic member 42 is brought into close contact with the lower surface of the specimen treatment chip 20 to eliminate a gap between the specimen treatment chip 20 and the heat generator 41. This allows heat of the heat generator 41 to be smoothly transmitted to the specimen treatment chip 20, so that temperature of the specimen treatment chip 20 can be accurately controlled. Simply moving the moving member 60 enables the connector 50 to be connected to the connecting port 22 and the heating unit 40 to be pressed on the specimen treatment chip 20. This enables a step of mounting the specimen treatment chip 20 to be simplified, so that operability when the specimen treatment chip 20 is mounted can be improved.

The connector 50 is not limited to a connection in which the connector 50 is pressed on the connecting port 22 to be connected thereto as described above. That is, the connector 50 may be directly connected to the connecting port 22, or the connector may be indirectly connected to the connecting port 22. When the connector 50 is indirectly connected to the connecting port 22, the connector 50 is connected to a reservoir for storing a reagent or the like, provided in the connecting port 22. Then, the connector 50 applies pressure to the reservoir so that a sample or the like is injected into the flow channel 21 from the reservoir through the connecting port 22, for example.

### <Description of Emulsion PCR Assay>

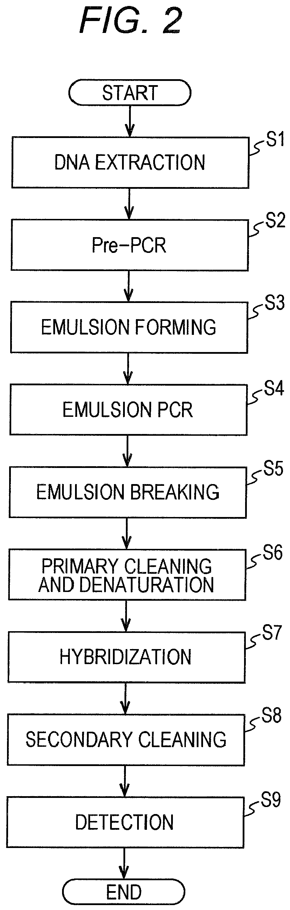

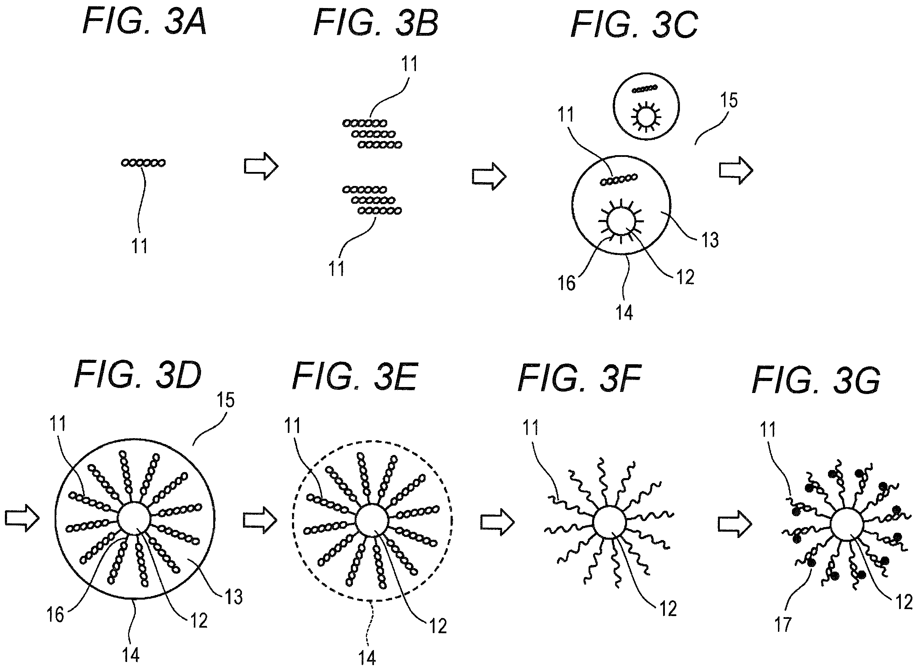

With reference to the flowchart illustrated in Fig. 2, an example of performing an emulsion PCR assay using the specimen treatment apparatus 10 and the specimen treatment chip 20, such as illustrated in Figs. 1A and 1B, will be described. All treatment steps illustrated in Fig. 2 may be performed by using the specimen treatment apparatus 10 and the specimen treatment chip 20, or some of the treatment steps illustrated in Fig. 2 may be performed. In this description, an illustration of each of Figs. 3A to 3G, showing progression, is appropriately referred.

At step S 1, DNA 11 is extracted from body fluid, blood, and the like, collected from a subject, by pretreatment as illustrated in Fig. 3A.

At step S2, the extracted DNA 11 is amplified by Pre-PCR treatment as illustrated in Fig. 3B. The Pre-PCR treatment is performed to preliminarily amplify the DNA 11 contained in an extraction liquid after the pretreatment to the extent that subsequent emulsion forming treatment is possible. In the Pre-PCR treatment, the extracted DNA 11, and a reagent for PCR amplification, containing polymerase and primer, are mixed, and the DNA 11 in the mixed liquid is amplified according to temperature control by a thermal cycler. In the temperature control by the thermal cycler, treatment of repeating multiple times a cycle in which the mixed liquid is varied to a plurality of different temperatures is performed. For example, when the mixed liquid has a temperature of 98°C, the DNA 11 is denaturalized to be a single strand, when the mixed liquid has a temperature of 72°C, the DNA 11 is extended, and when the mixed liquid has a temperature of 61 °C, the DNA 11 and the primer are bonded to each other.

At step S3, an emulsion containing the DNA 11, a magnetic particle 12, and a reagent 13 for amplification reaction, is formed as illustrated in Fig. 3C. The reagent 13 contains polymerase and the like. Specifically, at step S3, there are formed droplets 14 each containing a mixed liquid thereinside, the mixed liquid being formed of the DNA 11, the magnetic particle 12, and the reagent 13, and then a large number of the droplets 14 is dispersed into a dispersive medium 15. A primer 16 for nucleic acid amplification is applied to a surface of the magnetic particle 12. The droplet 14 is formed such that each of the magnetic particle 12 and a target DNA molecule is contained in the droplet 14 to the extent of about one piece. The dispersive medium 15 is immiscible to the mixed liquid. In this example, the mixed liquid is water-based, and the dispersive medium 15 is oil-based. The dispersive medium 15 is oil, for example.

At step S4, as emulsion PCR treatment, the DNA 11 is bonded to the primer 16 on the magnetic particle 12 and is amplified in each of the droplets 14 of the emulsion according to temperature control by the thermal cycler, as illustrated in Fig. 3D. In addition, the treatment of repeating multiple times a cycle in which the mixed liquid is varied to a plurality of different temperatures is performed according to the temperature control by the thermal cycler at step S4, as with step S2. For example, when the mixed liquid has a temperature of 98°C, the DNA 11 is denaturalized to be a single strand in the droplet 14, when the mixed liquid has a temperature of 72°C, the DNA 11 is extended in the droplet 14, and when the mixed liquid has a temperature of 61°C, the DNA 11 and the primer 16 are bonded to each other in the droplet 14. This causes the target DNA molecule to be amplified in each of the droplets 14.

At step S5, as emulsion breaking treatment, the droplet 14 is broken so that the magnetic particle 12 containing the amplified DNA 11 is extracted from the droplet 14 as illustrated in Fig. 3E. To break the droplet 14, one or more kinds of reagent containing alcohol, surface-active agent, and the like are used.

At step S6, as primary cleaning and denaturation treatment, the magnetic particle 12 extracted from the droplet 14 is cleaned in a primary BF separation step as illustrated in Fig. 3F. The primary BF separation step is a treatment step in which the magnetic particle 12 containing the amplified DNA 11 is passed through a cleaning liquid while being collected by a magnetic force so that unnecessary substances adhering to the magnetic particle 12 is removed. In the primary BF separation step, a cleaning liquid containing alcohol is used, for example. The alcohol removes an oil film on the magnetic particle 12. The alcohol also denaturalizes amplified double stranded DNA to a single strand.

At step S7, as hybridization treatment, the DNA 11 denaturalized to the single strand on the magnetic particle 12 is bonded to a marking substance 17 for detection as illustrated in Fig. 3G. The marking substance 17 is a fluorescent substance, for example. The marking substance 17 is designed to be specifically bonded to DNA 11 to be detected.

At step S8, as secondary cleaning treatment, the magnetic particle 12 bonded to the marking substance 17 is cleaned in a secondary BF separation step. The secondary BF separation step is performed by treatment similar to that of the primary BF separation step. In the secondary BF separation step, PBS, or phosphate buffered saline is used as a cleaning liquid, for example. The PBS removes an unreacted marking substance 17 that is not bonded to the DNA 11, or the marking substance 17 that is nonspecifically absorbed to the magnetic particle 12, for example.

At step S9, the DNA 11 is detected with a hybridized marking substance 17. The DNA 11 is detected with a flow cytometer, for example. In the flow cytometer, the magnetic particle 12 containing the DNA 11 bonded to the marking substance 17 flows through a flow cell, and the magnetic particle 12 is irradiated with a laser beam. Then, fluorescence emitted from the marking substance 17 by being irradiated with the laser beam is detected.

The DNA 11 may be detected by image processing. For example, the magnetic particles 12 each containing the DNA 11 bonded to the marking substance 17 are dispersed on a flat slide or in a flow channel, and the dispersed magnetic particles 12 are imaged by an imaging unit. The number of the magnetic particles 12 emitting fluorescent is counted on the basis of the imaged image.

### <Specific Structure of Embodiment 1>

When the emulsion PCR assay is performed as described above, a specific structure of the embodiment 1 described below is used. Structure of a specimen treatment apparatus 100 and a specimen treatment chip 200, being a specific structure of the embodiment 1, will be described below.

The specimen treatment apparatus 100 corresponds to the specimen treatment apparatus 10 of Fig. 1A. The specimen treatment chip 200 corresponds to the specimen treatment chip 20 of Fig. 1A. A placement part 160 corresponds to the placement part 30 of Fig. 1A. Heating units 170a and 170b each correspond to the heating unit 40 of Fig. 1A. Heat generators 171 and 173 each correspond to the heat generator 41 of Fig. 1A. Elastic members 172 and 174 each correspond to the elastic member 42 of Fig. 1A. A connector 140 corresponds to the connector 50 of Fig. 1A. A moving member 130 corresponds to the moving member 60 of Fig. 1A.

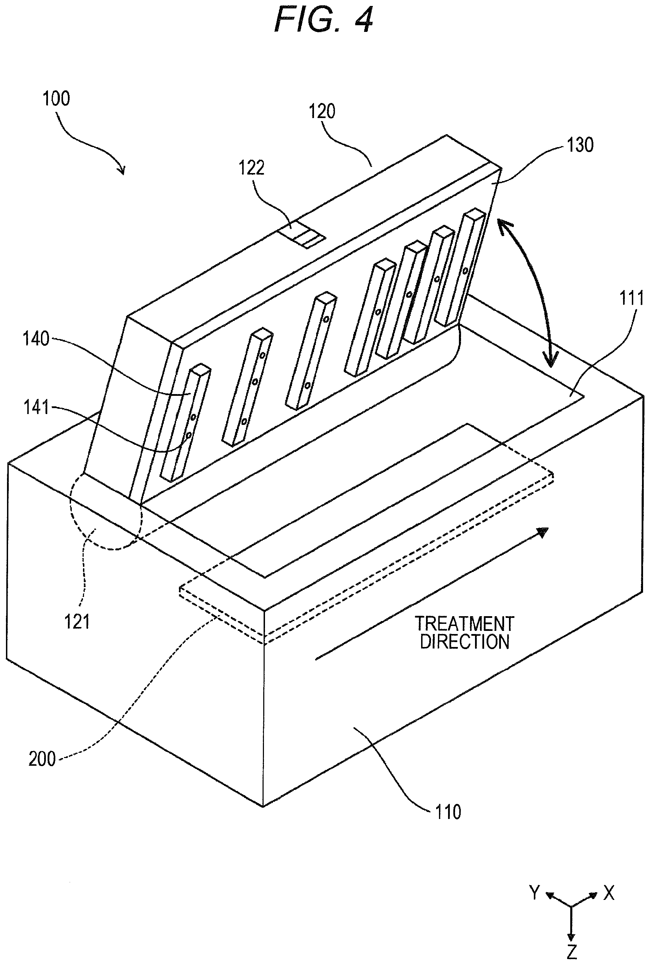

As illustrated in Fig. 4, the specimen treatment apparatus 100 is configured to be able to perform specimen treatment for performing genetic testing for an object component of nucleic acid such as DNA by using the specimen treatment chip 200. The specimen treatment chip 200 is used for nucleic acid amplification. At step S1 of Fig. 2, DNA is extracted from body fluid, blood, and the like, collected from a subject, by pretreatment. A specimen containing the extracted DNA is injected into the specimen treatment chip 200. Then, the specimen treatment apparatus 100 performs treatment at each of steps S2 to S7 of Fig. 2 by using the specimen treatment chip 200 into which the specimen is injected. The specimen treatment apparatus 100 may be configured to perform treatment at each of steps S1 to S9 of Fig. 2.

As illustrated in FIG. 4, the specimen treatment apparatus 100 includes a body 110, a lid part 120, a moving member 130, and a plurality of connectors 140.

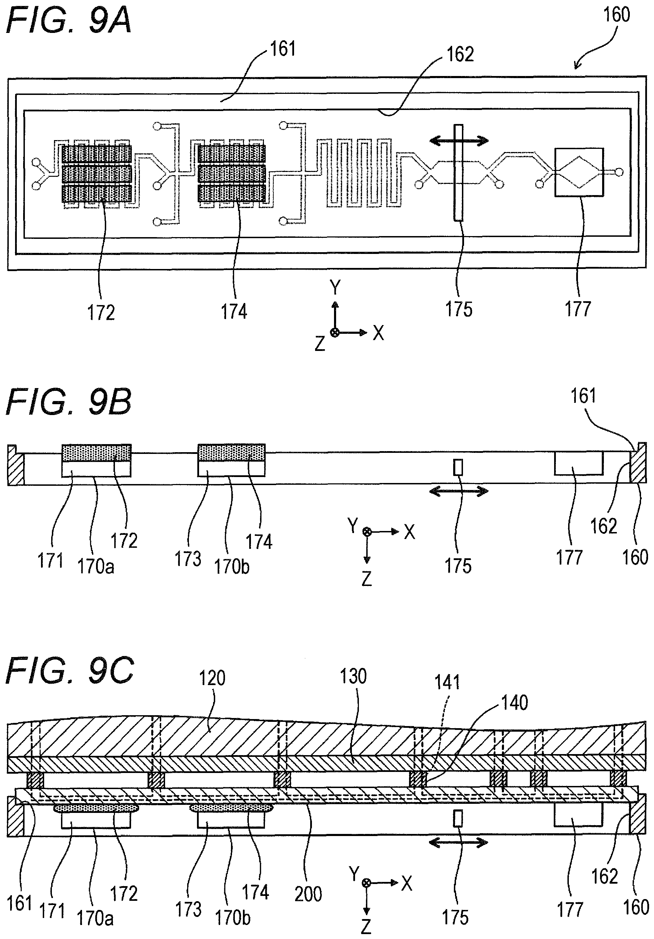

The body 110 provided in its upper surface with an opening 111. The lid part 120 is supported in the body 110 with a hinge 121 in an openable and closable manner. The lid part 120 is turned around the hinge 121 extending in the X-axis direction as indicated by the bold line arrow. When the lid part 120 is opened, the placement part 160 described below with reference to Fig. 9A is opened upward through the opening 111. This enables attaching and detaching of the specimen treatment chip 200 with respect to the placement part 160.

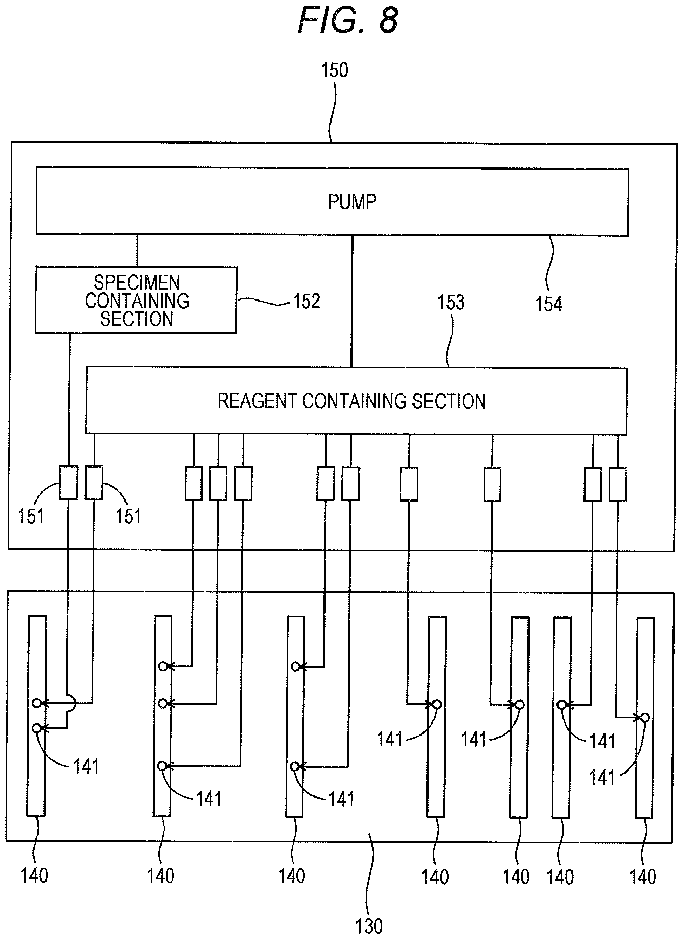

The moving member 130 is provided in a surface of the lid part 120, facing the opening 111 of the body 110, or a lower surface of the lid part 120. Seven connectors 140 are provided in a surface of the moving member 130, facing the opening 111, or a lower surface of the moving member 130. Each of the connectors 140 is provided with one or more holes 141 in place. The lid part 120 is provided in its inside with a liquid feeding pipe for allowing the hole 141 of the connector 140 to communicate with a valve 151 described below with reference to Fig. 8. When the moving member 130 is provided in the lid part 120 as described above, the connector 140 can be connected to the connecting port of the specimen treatment chip 200 and the elastic members 172 and 174 can press the specimen treatment chip 200, by only closing the lid part 120.

When the moving member 130 is provided in the lid part 120 as described above, simply closing the lid part 120 enables the connector 140 to be connected to the connecting port of the specimen treatment chip 200, and the elastic members 172 and 174 to press the specimen treatment chip 200.

The moving member 130 may be configured to be moved in conjunction with movement of the lid part 120 instead of being provided in the lower surface of the lid part 120. When the moving member 130 is moved in conjunction with movement of the lid part 120, an interlocking mechanism needs to be provided. Thus, it is desirable that the moving member 130 is directly provided in the lid part 120 as illustrated in Fig. 4. In the case of Fig. 4, no interlocking mechanism needs to be provided, so that the specimen treatment apparatus 100 can be simply formed. The moving member 130 may be formed integrally with the lid part 120. This case also enables the connector 140 to be connected to the connecting port of the specimen treatment chip 200 and the elastic members 172 and 174 to press the specimen treatment chip 200, by only closing the lid part 120 formed integrally with the moving member 130.

When the specimen treatment chip 200 is provided inside the body 110 and the lid part 120 is closed, a locking part 122 maintains the lid part 120 in a closed state. After that, treatment of a specimen is started in response to a start instruction by an operator. In the specimen treatment chip 200, a direction in which the treatment proceeds by allowing the specimen to flow is an X-axis positive direction.

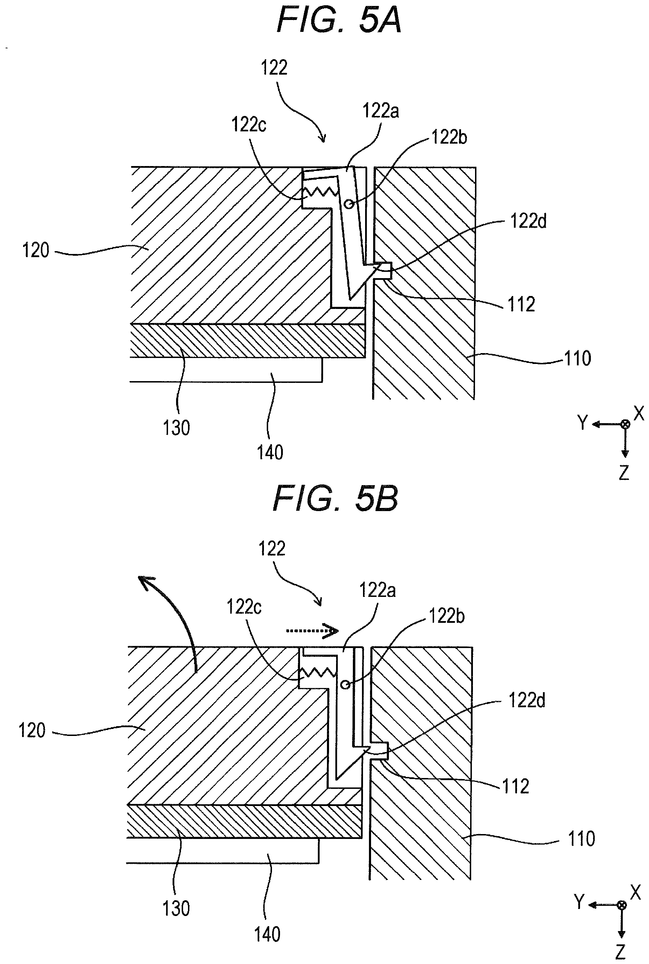

As illustrated in Fig. 5A, the locking part 122 includes a locking member 122a, a shaft member 122b, and a spring 122c. The shaft member 122b extends in the X-axis direction to be fixed to the lid part 120. The locking member 122a is supported by the shaft member 122b so as to be able to turn around the X-axis. The spring 122c connects a portion of the locking member 122a near its upper end to the lid part 120. The locking member 122a is urged by the spring 122c in a Y-axis positive direction. Near the lower end of the locking member 122a, a protrusion 122d is formed. A recessed portion 112 is formed in an inner surface of the body 110 on a Y-axis negative side. The lid part 120 is urged by a spring (not illustrated) in its opening direction.

As illustrated in Fig. 5A, when the lid part 120 is closed, the protrusion 122d is fitted into the recessed portion 112. This allows the lid part 120 to be maintained in a closed state. That is, the locking part 122 locks the lid part 120 and the moving member 130 by fitting the protrusion 122d into the recessed portion 112 to maintain a state where the connector 140 is connected to the connecting port and a state where the elastic members 172 and 174 are in close contact with the specimen treatment chip 200.

As illustrated in Fig. 5B, when the lid part 120 is opened, an operator slides an upper surface of the locking member 122a in a Y-axis negative direction as indicated by the dotted line arrow. This allows the protrusion 122d to move away from the recessed portion 112, so that the lid part 120 can turn in its opening direction. Then, the operator opens the lid part 120 as indicated by the bold line arrow while maintaining a state where the locking member 122a is slid.

The locking part 122 formed as described above causes a force of pressing the specimen treatment chip 200 applied by the connector 140 to be relatively weak when the locking part 122 locks the lid part 120 and the moving member 130. When the elastic members 172 and 174 are not provided on upper surfaces of the heat generators 171 and 173, respectively, unlike a case described below with reference to Figs. 9A to 9C, the force of pressing the specimen treatment chip 200 applied by the connector 140 needs to be increased to suitably transmit heat of each of the heat generators 171 and 173 to the specimen treatment chip 200. Unfortunately, in this case, the specimen treatment chip 20 may be deformed, and the deformation of the specimen treatment chip 20 may cause a flow channel in the specimen treatment chip 20 to be clogged. In addition, in this case, heat of each of the heat generators 171 and 173 is liable to cause the specimen treatment chip 200 to be deformed.

However, according to the embodiment 1, the elastic members 172 and 174 are provided on the upper surfaces of the heat generators 171 and 173, respectively, so that even a relatively weak force of pressing the specimen treatment chip 200 applied by the connector 140 enables heat of each of the heat generators 171 and 173 to be suitably transmitted to the specimen treatment chip 200. Thus, according to the embodiment 1, a mechanism for pressing the moving member 130 downward with a strong force does not need to be provided, so that the specimen treatment apparatus 100 can be simply formed. The specimen treatment chip 200 is not pressed with a strong force, so that deformation of the specimen treatment chip 200 and clogging of a flow channel of the specimen treatment chip 200 can be prevented.

The locking part 122 may be provided in the body 110, and the recessed portion into which the locking part 122 is fitted may be provided in the lid part 120. While the locking part 122 is provided in the lid part 120 only on its Y-axis negative side, the locking part 122 may be provided on an X-axis positive side and an X-axis negative side of the lid part 120.

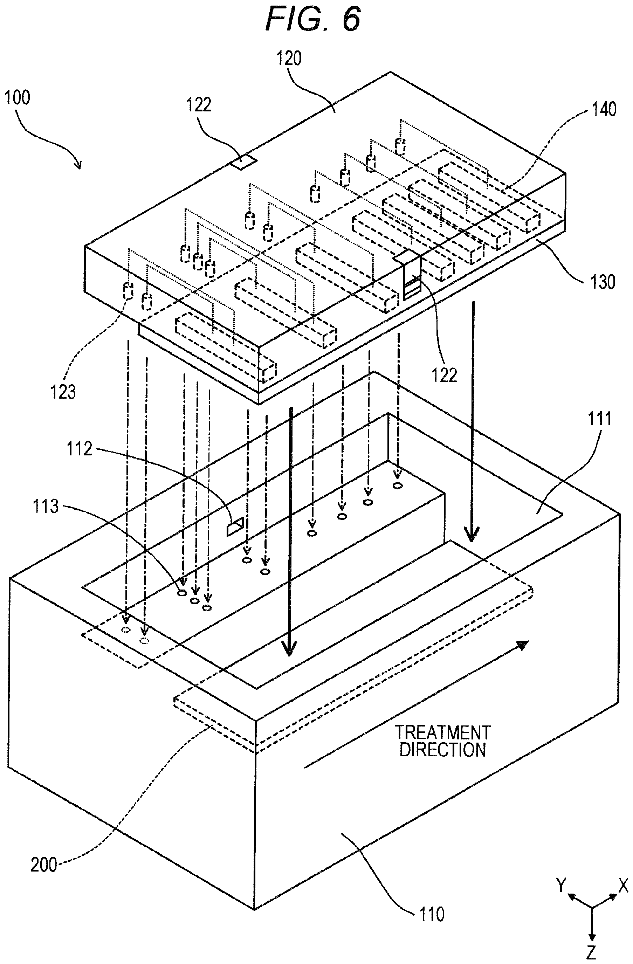

While the lid part 120 is supported in the body 110 with the hinge 121 in an openable and closable manner, besides this, the lid part 120 may be formed to be detachable to the body 110 as illustrated in Fig. 6.

As illustrated in Fig. 6, the moving member 130 is provided on a lower surface of the lid part 120 on its Y-axis negative side, and a plurality of tubular members 123 is provided on a lower surface of the lid part 120 on its Y-axis positive side. Inside the lid part 120, there is provided a liquid feeding pipe for allowing the hole 141 of the connector 140 to communicate with the tubular member 123 a as indicated by a dotted line. The locking part 122 is provided in the lid part 120 also on its Y-axis positive side. The lid part 120 is provided in its inside with a liquid feeding pipe for allowing the hole 141 of the connector 140 to communicate with the tubular member 123a as indicated by a dotted line. The holes 113 are disposed at respective positions corresponding to the tubular members 123 provided in the lid part 120. The body 110 is provided in its inside with a liquid feeding pipe for allowing the hole 113 to communicate with the valve 151 described below with reference to Fig. 8. The recessed portion 112 is formed also in a side surface inside the body 110 on its Y-axis positive side.

When the lid part 120 is fitted into the opening 111 from above, the two locking parts 122 provided in the lid part 120 are fitted into the two respective recessed portions 112 provided in the body 110 to maintain a closed state of the opening 111 with the lid part 120. At this time, the tubular members 123 in the lower surface of the lid part 120 are inserted into the corresponding holes 113 in the body 110, so that the hole 141 of the connector 140 communicates with the valve 151 described below. As with the case of Fig. 4, the connector 140 is connected to the connecting port of the specimen treatment chip 200, and the elastic members 172 and 174 described below press on the specimen treatment chip 200.

Even when the lid part 120 is provided in the body 110 in a detachable manner as illustrated in Fig. 6, simply fitting the lid part 120 into the opening 111 enables the connector 140 to be connected to the connecting port of the specimen treatment chip 200, and the elastic members 172 and 174 to press the specimen treatment chip 200.

Subsequently, structure of the specimen treatment chip 200, and structure inside the specimen treatment apparatus 100, will be described.

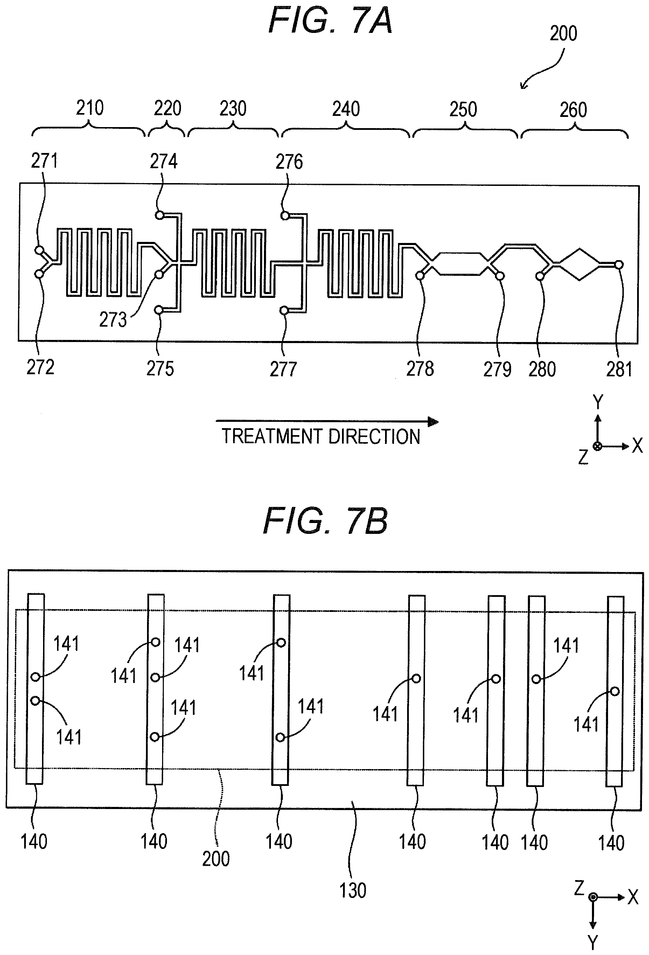

Fig. 7A illustrates a state of the specimen treatment chip 200 provided inside the specimen treatment apparatus 100, as viewed from above. As illustrated in Fig. 7A, the specimen treatment chip 200 includes flow channels 210 to 260, and connecting ports 271 to 281.

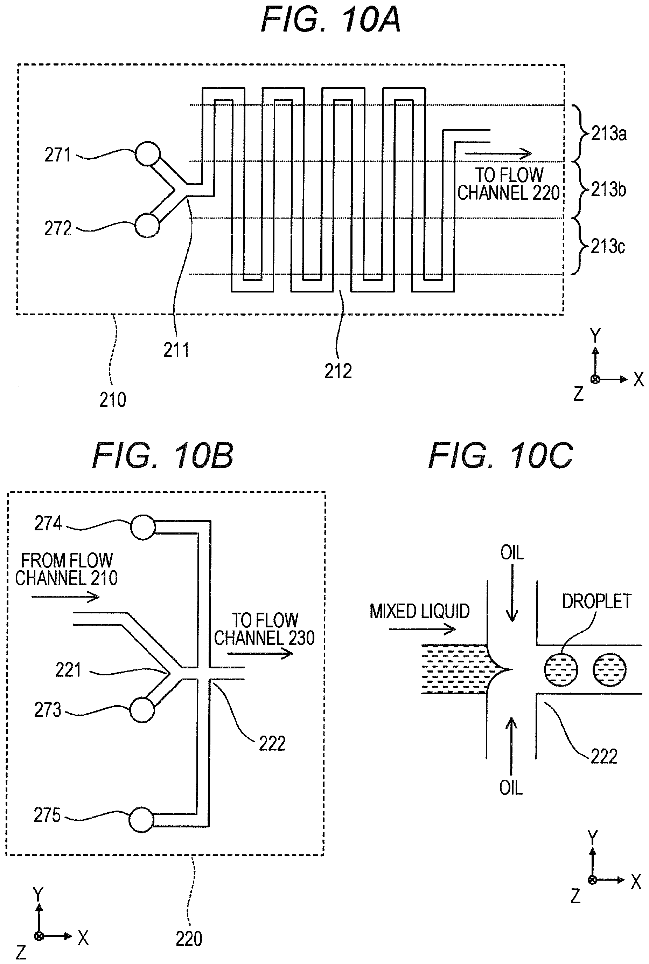

The flow channels 210 to 260 are provided in the specimen treatment chip 200 in order from its left end to right end. Treatment to be performed in each of the flow channels 210 to 260 will be described below with reference to Figs. 10A to 12B. The connecting ports 271 and 272 are provided at the same position in the X-axis direction. The connecting ports 273 to 275 are provided at the same position in the X-axis direction. The connecting ports 276 and 277 are provided at the same position in the X-axis direction. The specimen treatment chip 200 has an upper surface that is flat except portions where the connecting ports 271 to 281 are formed. The specimen treatment chip 200 has a lower surface that is flat.

The specimen treatment chip 200 is formed by bonding a planar substrate provided on its one side surface with grooves corresponding to the flow channels 210 to 260, and a planar substrate provided with holes corresponding to the connecting ports 271 to 281, to each other. When the two substrates are bonded to each other, the flow channels 210 to 260 are formed inside the specimen treatment chip 200. Then, the flow channels 210 to 260 communicate with the outside through the corresponding connecting ports 271 to 281.

Fig. 7B illustrates the moving member 130 in a state where the lid part 120 is closed, as viewed from below. Fig. 7B indicates a position corresponding to the specimen treatment chip 200 by a dotted line for convenience.

The connectors 140 are provided on the lower surface of the moving member 130 so as to align with the corresponding positions in the X-axis direction of the connecting ports 271 to 281 of the specimen treatment chip 200 placed inside the specimen treatment apparatus 100. The connector 140 at the left end has the two holes 141 that are at respective positions corresponding to the connecting ports 271 and 272. The second connector 140 from the left has the three holes 141 that are at respective positions corresponding to the connecting ports 273 to 275. The third connector 140 from the left has the two holes 141 that are at respective positions corresponding to the connecting ports 276 and 277. The hole 141 of each of the fourth connector 140 from the right, the third connector 140 from the right, the second connector 140 from the right, and the connector 140 at the right end, is at the corresponding one of positions corresponding to the connecting ports 278 to 281.

To meet various placements of the connecting ports provided in the upper surface of the specimen treatment chip 200, about the eight holes 141 may be provided in the one connector 140 so as to align in the Y-axis direction.

As illustrated in Fig. 8, the hole 141 of the connector 140 communicates with a liquid feeder 150. The liquid feeder 150 includes the valve 151, a specimen containing section 152, a reagent containing section 153, and a pump 154.

The valve 151 is an electromagnetic valve, for example. The valve 151 opens and closes a flow channel allowing the specimen treatment chip 200 and the specimen containing section 152 to communicate with each other by moving a plunger, so that a flow channel allowing the specimen treatment chip 200 and the reagent containing section 153 to communicate with each other is opened and closed.

The specimen containing section 152 contains a specimen containing DNA extracted by pretreatment. The reagent containing section 153 contains a reagent for PCR amplification, a reagent containing magnetic particles and a reagent for amplification reaction, a dispersive medium, a reagent for breaking a droplet, a cleaning liquid, and a reagent containing a marking substance, as a reagent for nucleic acid amplification. Specifically, the specimen containing section 152 is provided with a specimen container that contains a specimen. The reagent containing section 153 is provided with a reagent container that individually contains various reagents, a container that contains an unnecessary liquid discharged from the specimen treatment chip 200 in cleaning and denaturation treatment, and a container that contains a sample after treatment in the specimen treatment chip 200 is finished.

The specimen containing section 152 communicates with the hole 141 positioned on a Y-axis positive side of the connector 140 at the left end through the valve 151. The reagent containing section 153 communicates with the hole 141 positioned on the Y-axis negative side of the connector 140 at the left end, and the holes 141 of the six connectors 140 positioned on the right side, through the respective valves.

The pump 154 is a pressure pump that supplies air pressure, for example. The pump 154 may be a syringe pump or a diaphragm pump. The pump 154 applies pressure to the specimen containing section 152 and the reagent containing section 153. The pump 154 applies positive pressure to the specimen containing section 152 and the reagent containing section 153 to feed a predetermined liquid to a predetermined connecting port of the specimen treatment chip 200 from the specimen containing section 152 and the reagent containing section 153. The pump 154 applies negative pressure to the reagent containing section 153 to feed a liquid to the reagent containing section 153 from a predetermined connecting port of the specimen treatment chip 200.

As illustrated in Fig. 9A, the placement part 160 has a rectangular shape extending in the X-axis direction as viewed vertically downward. As viewed vertically downward, the placement part 160 is provided at its middle with a recessed portion 161 smaller than an outline of the placement part 160. The recessed portion 161 has a bottom surface that is a supporting surface for supporting the specimen treatment chip 200 upward. The placement part 160 is also provided at its middle with a hole 162 that is slightly smaller than the recessed portion 161. The recessed portion 161 has a substantially identical shape to that of the specimen treatment chip 200 in an XY-plane. The hole 162 vertically passes through the placement part 160. Fig. 9A illustrates a flow channel of the specimen treatment chip 200 placed in the placement part 160 by a dotted line for convenience.

As illustrated in Figs. 9A and 9B, the heating units 170a and 170b are positioned inside the hole 162 in the XY-plane. The heating unit 170a includes the heat generator 171 and the elastic member 172, and the heating unit 170b includes the heat generator 173 and the elastic member 174. The heat generators 171 and 173 are provided inside the specimen treatment apparatus 100. The elastic members 172 and 174 are respectively provided on upper surfaces of the heat generators 171 and 173, the upper surfaces each being a heat generating surface thereof. The three heating units 170a are disposed at respective positions corresponding to a flow channel 210 while aligning in the Y-axis direction. The three heating units 170b are disposed at respective positions corresponding to a flow channel 230 while aligning in the Y-axis direction. Fig. 9B illustrates the heating units 170a and 170b positioned on a Y-axis negative side. Each of upper surfaces of the heat generators 171 and 173 is positioned below a lower surface of the recessed portion 161, and each of upper surfaces of the elastic members 172 and 174 is positioned above the lower surface of the recessed portion 161.

One elastic member 172 may be set on upper surfaces of three respective heat generators 171 so as to extend over the upper surfaces of the three respective heat generators 171. Likewise, one elastic member 174 may be set on upper surfaces of three respective heat generators 173 so as to extend over the upper surfaces of the three respective heat generators 173. In this case, a temperature of a flow channel of the specimen treatment chip 200 positioned above the heat generators 171 and 173 tends to be difficult to be individually controlled. Thus, as illustrated in Figs. 9A and 9B, it is desirable that the elastic member 172 is provided on each of three heat generators 171, and that the elastic member 174 is provided on each of three heat generators 173.

Three elastic moduli of the three respective elastic members 172 may be different from each other in accordance with temperatures set to the corresponding flow channels. Likewise, three elastic moduli of the three respective elastic members 174 may be different from each other in accordance with temperatures set to the corresponding flow channels.

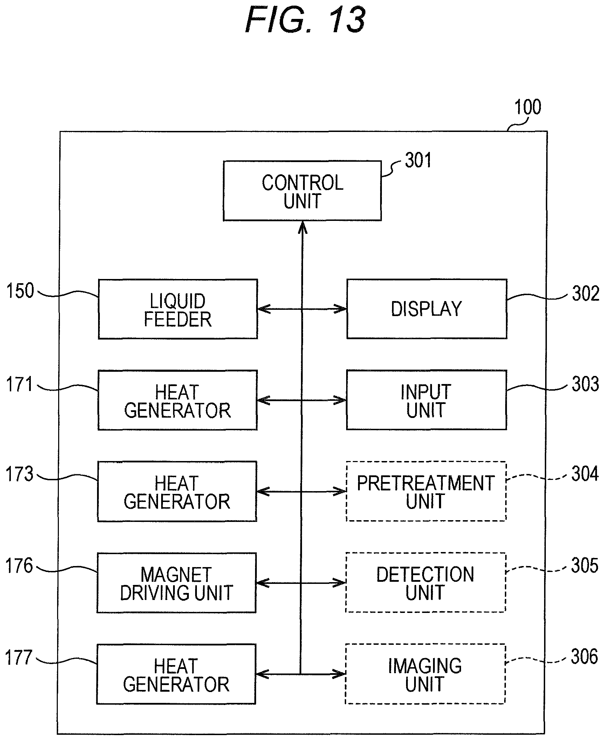

As illustrated in Figs. 9A and 9B, a magnet 175 and a heat generator 177 are positioned inside the hole 162 in the XY-plane. The magnet 175 is disposed at a position corresponding to a flow channel 250. The magnet 175 has a vertical magnetization direction. The magnet 175 is movable in the X-axis direction as indicated by the bold arrow by using a magnet driving unit 176 shown in Fig. 13. The heat generator 177 is disposed at a position corresponding to the flow channel 260. The heat generator 177 has an upper surface serving as a heat generating surface. The upper surface of the heat generator 177 is positioned at a height that is substantially identical to that of the lower surface of the recessed portion 161. Heating for the flow channel 260 does not need to be strictly performed as compared with heating for each of the flow channels 210 and 230, so that no elastic member is provided on the upper surface of the heat generator 177.

As illustrated in Fig. 9C, when the specimen treatment chip 200 is placed in the placement part 160 and the lid part 120 is closed to move the moving member 130 downward, the connector 140 is pressed on the specimen treatment chip 200. At this time, a lower surface of the specimen treatment chip 200 is brought into contact with the lower surface of the recessed portion 161 to determine a vertical position of the specimen treatment chip 200. The specimen treatment chip 200 is fitted into the recessed portion 161 to determine a position of the specimen treatment chip 200 in the horizontal plane.

When the moving member 130 is moved downward as described above, a hole 141 of each of the connectors 140 is connected to the corresponding one of connecting ports of the specimen treatment chip 200. Then, the lower surface of the specimen treatment chip 200 is pressed on the elastic members 172 and 174, so that the elastic members 172 and 174 are elastically deformed to be brought into close contact with the lower surface of the specimen treatment chip 200. Thus, an effect similar to that of the case of Figs. 1A and 1B can be achieved. That is, a gap between the specimen treatment chip 200, and the heat generators 171 and 173, is eliminated, so that temperature control for the specimen treatment chip 200 can be accurately performed. Simply moving the moving member 130 enables the connector 140 to be connected to a connecting port, and then enables the heating units 170a and 170b, specifically the elastic members 172 and 174, to be pressed on the specimen treatment chip 200. This enables improvement in operability when the specimen treatment chip 200 is mounted.

When the connectors 140 are connected to the corresponding connecting ports of the specimen treatment chip 200 and the lower surface of the specimen treatment chip 200 is brought into close contact with the elastic members 172 and 174 as illustrated in Fig. 9C, treatment of a specimen using the specimen treatment chip 200 is started. When the treatment of the specimen is started, the specimen absorbed from the specimen containing section 152 is injected into the specimen treatment chip 200 through the hole 141 on the Y-axis positive side of the connector 140 positioned at the left end, and a reagent absorbed from the reagent containing section 153 is injected into the specimen treatment chip 200 through the holes 141 of the respective connectors 140. When the specimen and the reagent are injected into a flow channel of the specimen treatment chip 200 from the plurality of connectors 140 as described above, treatment of nucleic acid amplification described below can be smoothly performed.

Subsequently, structure of each of the flow channels 210 to 260 of the specimen treatment chip 200 will be described according to a flow of a liquid containing nucleic acid.

As illustrated in Fig. 10A, Pre-PCR treatment corresponding to step S2 of Fig. 2 is performed in the flow channel 210. The flow channel 210 includes an intersection 211 and a meander flow channel 212. Flow channels communicating with the respective connecting ports 271 and 272 are formed on the left side of the intersection 211. The meander flow channel 212 is positioned downstream of the intersection 211, or on an X-axis positive side, and a downstream side of the meander flow channel 212 communicates with a flow channel 220. From the connecting port 271, a specimen containing DNA extracted by pretreatment is injected. From the connecting port 272, a reagent for PCR amplification is injected. The specimen and the reagent for PCR amplification are mixed at the intersection 211.

The meander flow channel 212 meanders several times suitable for the number of thermal cycles, in the Y-axis direction with a predetermined amplitude. A first region 213a is set on a Y-axis positive side across a middle portion of the meander, a second region 213b is set in the middle portion of the meander, and a third region 213c is set on a Y-axis negative side across the middle portion of the meander. The heating unit 170a is disposed in each of the first region 213a, the second region 213b, and the third region 213c.

Portions of the meander flow channel 212 corresponding to the first region 213a, the second region 213b, and the third region 213c are flow channels for processes of amplification, extension, and bonding of nucleic acid, respectively. The heat generator 171 disposed in each of the first region 213a, the second region 213b, and the third region 213c, is controlled so as to have a temperature of the corresponding one of processes of amplification, extension, and bonding of nucleic acid.

For example, the heat generator 171 disposed in the first region 213a is controlled so that a mixed liquid flowing through the first region 213a has a temperature of 98°C. The heat generator 171 disposed in the second region 213b is controlled so that the mixed liquid flowing through the second region 213b has a temperature of 72°C. The heat generator 171 disposed in the third region 213c is controlled so that the mixed liquid flowing through the third region 213c has a temperature of 61°C. As a result, DNA is denaturalized to be a single strand in the first region 213a, the DNA is extended in the second region 213b, and the DNA and a primer are bonded to each other in the third region 213c. The mixed liquid to which thermal cycle treatment is applied in the meander flow channel 212 is fed to the flow channel 220 on a downstream side.

When the heating units 170a are disposed in association with different regions from each other of the flow channel formed in the specimen treatment chip 200, the different regions of the flow channel can be heated at respective different temperatures from each other. As a result, temperature control can be finely performed. Then, temperatures appropriate for respective processes of amplification, extension, and bonding of nucleic acid can be applied to the corresponding flow channel portions, so that temperature control during the nucleic acid amplification can be performed with high accuracy and reliably. Specifically, when the meander flow channel 212 is formed and the heating unit 170a is disposed at each of the three regions as illustrated in Fig. 10A, three temperatures can be applied to a mixed liquid by only allowing the mixed liquid to flow through the meander flow channel 212. This enables thermal cycle treatment of a desired number of cycles to be easily performed.

One heat generator 171 may be provided so as to extend over the first region 213a, the second region 213b, and the third region 213c, and one elastic member may be provided on an upper surface of the one heat generator 171. In this case, the heat generator 171 is controlled so as to have a temperature appropriate for each of the processes of amplification, extension, and bonding of nucleic acid.

As illustrated in Fig. 10B, emulsion forming treatment corresponding to step S3 of Fig. 2 is performed in the flow channel 220. The flow channel 220 includes intersections 221 and 222. Flow channels communicating with the flow channel 210 and a connecting port 273 are formed on the left side of the intersection 221. From the connecting port 273, a reagent containing a magnetic particle and a reagent for amplification reaction is injected. The mixed liquid fed through the flow channel 210 and the reagent injected from the connecting port 273 are mixed at the intersection 221.

The intersection 222 positioned downstream of the intersection 221. Flow channels communicating with connecting ports 274 and 275 are respectively formed on a Y-axis positive side and a Y-axis negative side of the intersection 221. A downstream side of the intersection 222 communicates with the flow channel 230. From the connecting ports 274 and 275, a dispersive medium is injected. The dispersive medium is oil for forming an emulsion, for example. The mixed liquid fed rightward from the intersection 221 and the oil injected from the connecting ports 274 and 275 are mixed at the intersection 222. At this time, a flow of the oil applies a shear force to a flow of the mixed liquid fed from the intersection 221 as illustrated in Fig. 10C. This enables droplets of the mixed liquid fed from the intersection 221 to be efficiently formed. The mixed liquid containing the droplets formed at the intersection 222 is fed to the flow channel 230 on the downstream side.

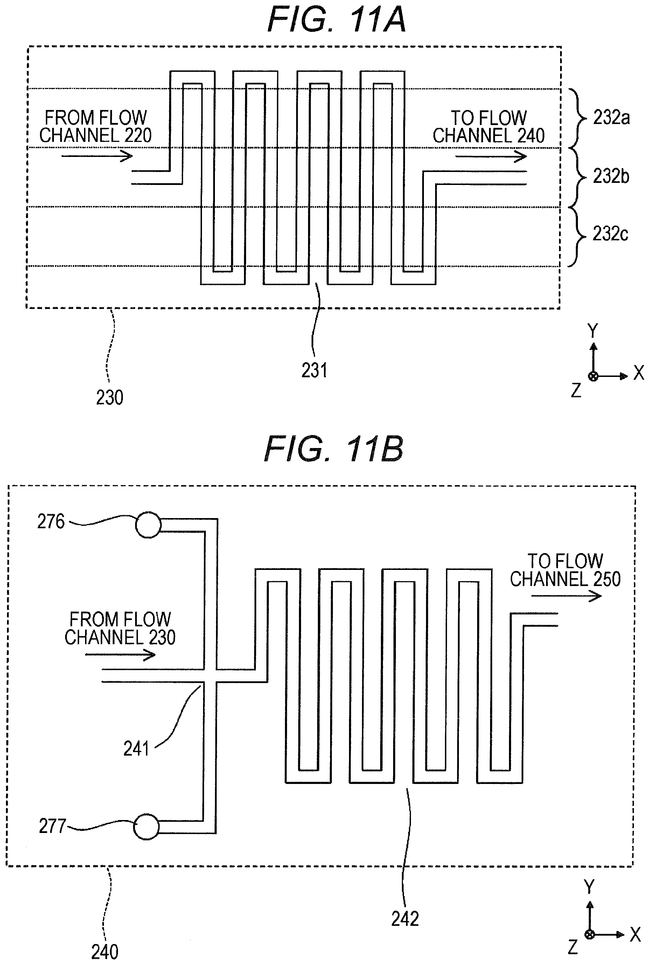

As illustrated in Fig. 11A, emulsion PCR treatment corresponding to step S4 of Fig. 2 is performed in the flow channel 230. The flow channel 230 includes a meander flow channel 231 similar to the meander flow channel 212 of Fig. 10A. An upstream side of the meander flow channel 231 communicates with the flow channel 220, and a downstream side of the meander flow channel 231 communicates with a flow channel 240.

The meander flow channel 231 meanders several times suitable for the number of thermal cycles, in the Y-axis direction with a predetermined amplitude. A first region 232a is set on a Y-axis positive side across a middle portion of the meander, a second region 232b is set in the middle portion of the meander, and a third region 232c is set on a Y-axis negative side across the middle portion of the meander. The heating unit 170b is disposed in each of the first region 232a, the second region 232b, and the third region 232c.

Portions of the meander flow channel 231 corresponding to the first region 232a, the second region 232b, and the third region 232c are flow channels for processes of amplification, extension, and bonding of nucleic acid, respectively. The heat generator 173 disposed in each of the first region 232a, the second region 232b, and the third region 232c, is controlled so as to have a temperature of the corresponding one of processes of amplification, extension, and bonding of nucleic acid, as with control of the three heat generators 171 disposed in the flow channel 210.

For example, the heat generator 173 disposed in the first region 232a is controlled so that a mixed liquid flowing through the first region 232a has a temperature of 98°C. The heat generator 173 disposed in the second region 232b is controlled so that the mixed liquid flowing through the second region 232b has a temperature of 72°C. The heat generator 173 disposed in the third region 232c is controlled so that the mixed liquid flowing through the third region 232c has a temperature of 61°C. As a result, DNA is denaturalized to be a single strand in the first region 232a, the DNA is extended in the second region 232b, and the DNA and a primer are bonded to each other in the third region 232c. When the meander flow channel 231 is formed as described above, an effect similar to that of the meander flow channel 212 of the flow channel 210 can be achieved. The mixed liquid to which thermal cycle treatment is applied in the meander flow channel 231 is fed to the flow channel 240 on the downstream side.

As illustrated in Fig. 11B, emulsion breaking treatment corresponding to step S5 of Fig. 2 is performed in the flow channel 240. The flow channel 240 includes an intersection 241 and a meander flow channel 242. A flow channel communicating with the flow channel 230 is formed on the left side of the intersection 241. Flow channels communicating with connecting ports 276 and 277 are respectively formed on a Y-axis positive side and a Y-axis negative side of the intersection 241. A downstream side of the intersection 241 communicates with the flow meander channel 242. From the connecting ports 276 and 277, a reagent for breaking a droplet is injected. The mixed liquid fed through the flow channel 230 and the reagent injected from the connecting ports 276 and 277 are mixed at the intersection 241.

The meander flow channel 242 meanders in the Y-axis direction with a predetermined amplitude. Droplets contained in the mixed liquid fed to the meander flow channel 242 are reliably mixed with the reagent for breaking a droplet during a process of flowing of the mixed liquid through the meander flow channel 242. As a result, the droplets are broken in the mixed liquid flowing through the meander flow channel 242, and a magnetic particle in each of the droplets is extracted. The mixed liquid containing the magnetic particle extracted from the each of the droplets in the meander flow channel 242 is fed to the flow channel 250 on the downstream side.

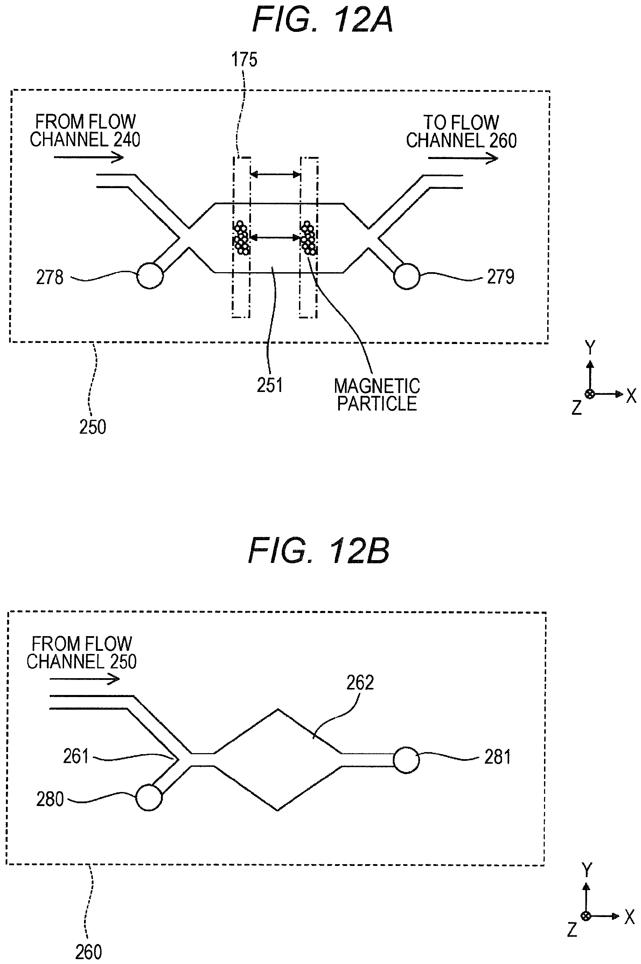

As illustrated in Fig. 12A, primary cleaning and denaturation treatment corresponding to step S6 of Fig. 2 are performed in the flow channel 250. The flow channel 250 includes a reservoir 251. Flow channels communicating with the flow channel 240 and the connecting port 278 are formed on the left side of the reservoir 251. From the connecting port 278, a cleaning liquid for primary cleaning is injected. Flow channels communicating with the flow channel 260 and the connecting port 279 are formed on the right side of the reservoir 251. From the connecting port 279, an unnecessary liquid in the reservoir 251 is discharged.

As illustrated in Fig. 12A by a dashed-dotted line, the magnet 175 is disposed below the reservoir 251, or on a Z-axis positive side. The magnet 175 is reciprocated in an X-axis direction by the magnet driving unit 176 shown in Fig. 13. In the primary cleaning and the denaturation treatment, the cleaning liquid is caused to continuously flow to the connecting port 279 from the connecting port 278 through the reservoir 251. Then, magnetic particles contained in the mixed liquid fed through the flow channel 240 are collected by the magnet 175, and the magnetic particles are cleaned in the cleaning liquid with reciprocation of the magnet 175. As a result, an oil film on the magnetic particle is removed to denaturalize amplified double stranded DNA to a single strand. When the primary cleaning and the denaturation treatment are finished, the magnetic particles are fed to the flow channel 260 on a downstream side.

As illustrated in Fig. 12B, hybridization treatment corresponding to step S7 of Fig. 2 is performed in the flow channel 260. The flow channel 260 includes an intersection 261 and a reservoir 262. Flow channels communicating with the flow channel 250 and a connecting port 280 are formed on the left side of the intersection 261. From the connecting port 280, a reagent containing a marking substance is injected. A downstream side of the intersection 261 communicates with the reservoir 262. A flow channel communicating with the connecting port 281 is formed downstream of reservoir 262.

The heat generator 177 is disposed below the reservoir 262, or on a Z-axis positive side. The magnetic particles fed through the flow channel 250 are mixed with the reagent containing a marking substance at the intersection 261 and the reservoir 262. The heat generator 177 performs a thermal cycle for the magnetic particles stored in the reservoir 262 and the mixed liquid containing the marking substance. This causes target DNA on the magnetic particle and the marking substance to be bonded to each other. When the hybridization treatment is finished, the mixed liquid in the reservoir 262 is fed into the connecting port 281 to be recovered in a container in the reagent containing section 153. Then, the treatment using the specimen treatment apparatus 100 and the specimen treatment chip 200 is finished.

While treatment of each of steps S2 to S7 of Fig. 2 is performed by using the specimen treatment apparatus 100 and the specimen treatment chip 200 in the specific structure of the embodiment 1, steps S1 and S8 of Fig. 2 may be further performed. In this case, a flow channel for performing DNA extraction treatment corresponding to step S1 of Fig. 2 is provided upstream of the flow channel 210 of the specimen treatment chip 200, and a flow channel for performing secondary cleaning treatment corresponding to step S8 of Fig. 2 is provided downstream of the flow channel 260 of the specimen treatment chip 200. Then, each of the connectors 140 is provided on the lower surface of the moving member 130 so as to align with the corresponding one of a flow channel for performing the DNA extraction treatment and a flow channel for performing the secondary cleaning treatment.

As illustrated in Fig. 13, the specimen treatment apparatus 100 includes the liquid feeder 150, the three heat generators 171, the three heat generators 173, and the heat generator 177, as described above. The specimen treatment apparatus 100 includes the magnet driving unit 176, a control unit 301, a display 302, and an input unit 303. The magnet driving unit 176 moves the magnet 175 in the X-axis direction as described above.

The control unit 301 includes an arithmetic processor and memory. The arithmetic processor is composed of a CPU, an MPU, or the like, for example. The memory is composed of a flash memory, a hard disk, or the like, for example. The control unit 301 receives a signal from each unit of the specimen treatment apparatus 100 to control each unit of the specimen treatment apparatus 100. The display 302 and the input unit 303 are provided in a side surface portion of the body 110, an upper surface portion of the lid part 120, or the like, for example. The display 302 is composed of a liquid crystal panel, or the like, for example. The input unit 303 is composed of a button, a touch panel, or the like, for example.

When treatment of each of steps S1 to S9 of Fig. 2 is performed by using the specimen treatment apparatus 100 and the specimen treatment chip 200, the specimen treatment apparatus 100 may further include a pretreatment unit 304, and a detection unit 305, as indicated by a broken line in Fig. 13. The pretreatment unit 304 performs extract treatment of DNA of step S1. The detection unit 305 includes a flow cytometer, for example. The detection unit 305 applies detection treatment of step S9 to a magnetic particle containing a marking substance after the secondary cleaning treatment of step S8 by using the flow cytometer. In the flow cytometer, the magnetic particle containing DNA bonded to the marking substance flows into a flow cell, and the magnetic particle is irradiated with a laser beam. Then, target DNA is detected on the basis of fluorescence emitted from the marking substance 17 by receiving the laser beam.

The specimen treatment apparatus 100 may further include an imaging unit 306 as indicated by a broken line in Fig. 13. The imaging unit 306 images the magnetic particle containing the marking substance after the secondary cleaning treatment of step S8. The imaged image is analyzed by the control unit 301.

### <Verification of Heating through Elastic Member>

Verification of a heat generator that is provided, on its heat generating surface, with an elastic member, by which temperature control of a specimen treatment chip can be accurately performed, the verification being conducted by the inventors and others, will be described.

In the above verification, a micro fluid chip made of a cycloolefin polymer, provided with a meander flow channel (made by Microfluidic ChipShop GmbH, 08-0472-0061, t = 1.675 mm) was used as a specimen treatment chip. As a heat generator, a heater block with a length of 65.5 mm in its longitudinal direction, and a length of 6.05 mm in its lateral direction (made by Microfluidic ChipShop GmbH, 08-0495-000-00) was used.

Three heat generators were disposed while aligning in a lateral direction of the generator, like the heat generators 171 and 173 illustrated in Figs. 9A and 9B. Each of the three heat generators is disposed in the corresponding one of regions for denaturation treatment, extension treatment, and bonding treatment, like the three regions illustrated in Figs. 10A and 11A. Temperatures in respective regions for the denaturation treatment, the extension treatment, and the bonding treatment, were set at 98°C, 72°C, and 61°C, respectively. As an elastic member, a silicone rubber part (made of Niigata Co., Ltd., a thickness of 1 mm) was used. The elastic member was provided for each of the three heat generators. The elastic member had a size identical to a heat generating surface of the heat generator.

In a verification example 1, a specimen treatment chip was directly mounded on each of three heat generators, and the specimen treatment chip was simply fixed to prevent displacement thereof. In a verification example 2, a cover glass is provided on a heat generator to provide a gap between the heat generator and a specimen treatment chip. As the cover glass, a cover glass with a thickness of 0.12 mm to 0.17 mm (CO18181, made by Matsunami Glass Ind., Ltd.) was used. In the verification example 2, a specimen treatment chip was mounted on the cover glass that was mounted on a heat generator, and the specimen treatment chip was simply fixed to prevent displacement thereof. In a verification example 3, after the cover glass was mounted on a heat generator, an elastic member was mounted on each heat generator, as with the verification example 2, and a specimen treatment was further mounted on the elastic member. Then the specimen treatment chip was simply fixed to prevent displacement thereof.

In each of the verification examples 1 to 3, after a specimen treatment chip is placed, heating was started by using three heat generators. After elapse of five minutes after the heating was started, temperature of each region was measured with a thermographic camera (InfRec R500, made by Avionics Co., Ltd).

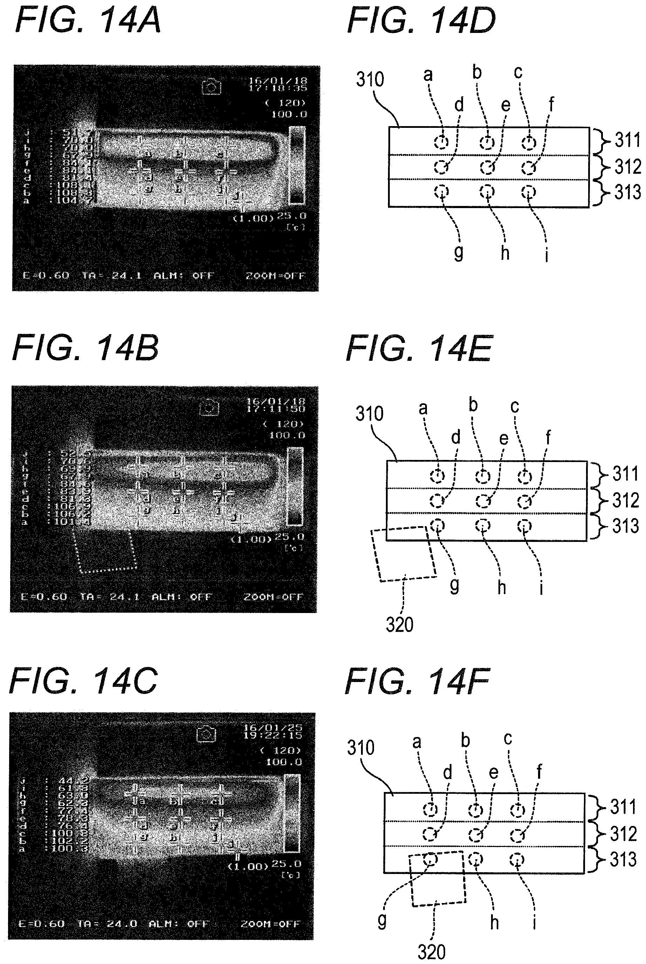

Figs. 14A to 14C are photographs respectively showing temperature distributions of specimen treatment chips of verification examples 1 to 3, acquired per one measurement. Figs. 14A to 14C are gray scale images acquired from a color image, for convenience. Figs. 14D to 14F are illustrations schematically showing states of Figs. 14A to 14C, respectively. Figs. 14D to 14F respectively illustrate a position of a specimen treatment chip 310 used in each verification by a solid line. Figs. 14D to 14F respectively illustrate regions 311 to 313 corresponding to regions for denaturation treatment, extension treatment, and bonding treatment, respectively. Figs. 14E and 14F respectively illustrate a position of a cover glass 320 used in each verification, by a broken line. As illustrated in Figs. 14E and 14F, the cover glass 320 was disposed near the left end of the specimen treatment chip 310 in the region 313 in each of the verification examples 2 and 3.

As illustrated in Figs. 14A to 14F, a temperature of a specimen treatment chip at each of positions a to i was measured in each of the verification examples 1 to 3. The positions a, b, and c are included in the region 311 for the denaturation treatment. The positions d, e, and f are included in the region 312 for the extension treatment. The positions g, h, and I are included in the region 313 for the bonding treatment. In each of the verification examples 1 to 3, a temperature of each of the positions a to i was acquired per one measurement. That is, temperatures at three respective positions in each the regions 311 to 313 were acquired per one measurement. Then, the measurement was performed three times in total, so that nine temperatures were acquired for each of the regions 311 to 313. In each of the verification examples, an average value of temperatures in each of the regions 311 to 313 and a CV value of the temperatures in each of the regions 311 to 313 were calculated from the nine temperatures acquired in each of the regions 311 to 313.

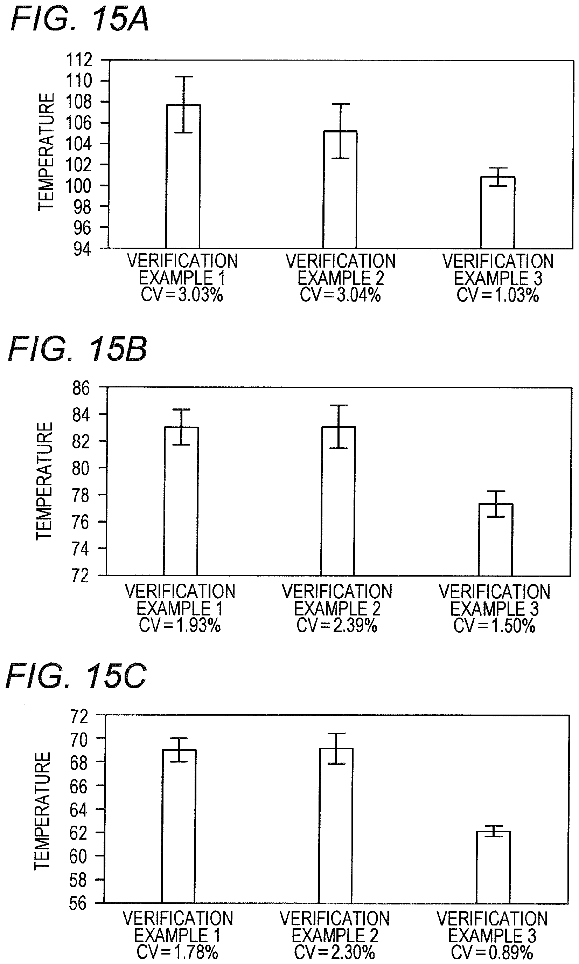

As illustrated in Fig. 15A, CV values of the verification examples 1 to 3 were 3.03%, 3.04%, and 1.03%, respectively, in the region 311 for denaturation treatment. As illustrated in Fig. 15B, CV values of the verification examples 1 to 3 were 1.93%, 2.39%, and 1.50%, respectively, in the region 312 for extension treatment. As illustrated in Fig. 15C, CV values of the verification examples 1 to 3 were 1.78%, 2.30%, and 0.89%, respectively, in the region 313 for bonding treatment.

In the case of the verification example 1, a slight gap is caused between the specimen treatment chip and the heat generator, and in the case of the verification example 2, a larger gap is caused between the specimen treatment chip and the heat generator due to the cover glass. This causes each of the verification examples 1 and 2 to have the CV values being high in any regions. Meanwhile, in the case of the verification example 3, while the cover glass is interposed, a gap is less likely to be caused between the specimen treatment chip and the heat generator due to the elastic member. This causes the verification example 3 to have the CV values being low in any regions. As described above, in the case of the verification example 3, it can be said that variation in temperature can be reduced in each region of a specimen treatment chip to allow temperature control for the specimen treatment chip to be accurately performed. Thus, it can be said that when the elastic member 42 is provided between the specimen treatment chip 20 and the heat generator 41 like the embodiment 1, temperature control for the specimen treatment chip 20 can be accurately performed.

In the case of the verification example 3, temperature of the heat generator is transmitted to the specimen treatment chip through the elastic member, so that temperature of the heat generator is less likely to be transmitted to the specimen treatment chip as compared with the verification examples 1 and 2 without using the elastic member. As a result, temperature of the verification example 3 is lower than that of each of the verification examples 1 and, as illustrated in Figs. 15A to 15C. However, even in the case of the verification example 3, when temperature of the heat generator is set higher, temperature of the specimen treatment chip can be set to a level equivalent to that of each of the verification examples 1 and 2.

### <Embodiment 2>

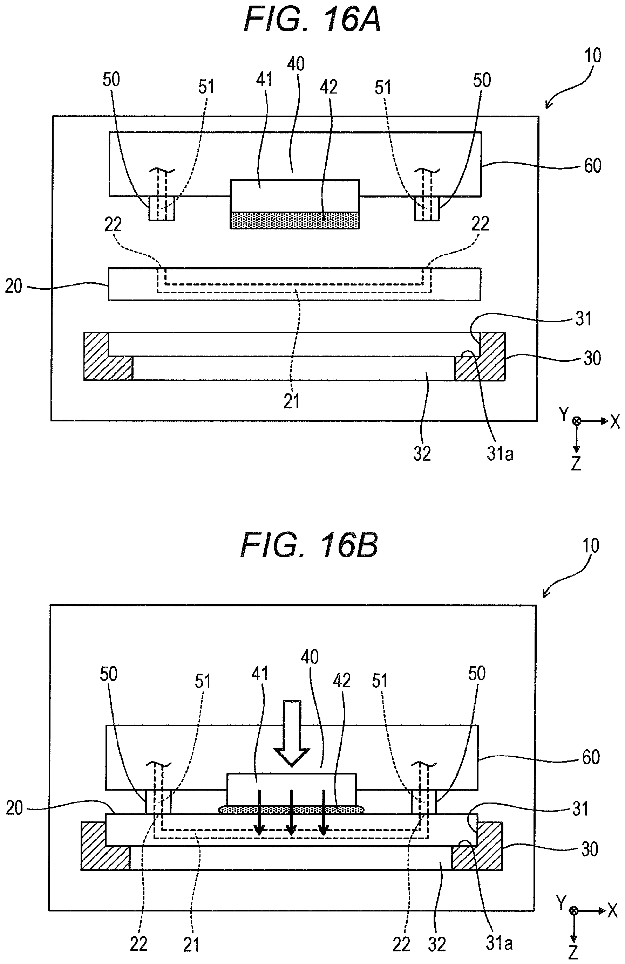

As illustrated in Figs. 16A and 16B, in an embodiment 2, a heating unit 40 is disposed on the same side as a connector 50 with respect to a specimen treatment chip 20 placed in a placement part 30. That is, in the embodiment 2, the heating unit 40 is provided in a moving member 60. Another structure of the embodiment 2 is similar to that of the embodiment 1.

As illustrated in Fig. 16A, a heat generator 41 is provided in a lower surface of the moving member 60, and a lower surface of the heat generator 41 serves as a heat generating surface. An elastic member 42 is provided on the lower surface of the heat generator 41. The lower surface of the heat generator 41 is positioned above a lower surface of the connector 50, and a lower surface of the elastic member 42 is positioned below the lower surface of the connector 50.

When the moving member 60 is moved downward from a state of Fig. 16A, the connector 50 is connected to a connecting port 22, and the heating unit 40, specifically the elastic member 42, is pressed on the specimen treatment chip 20, as illustrated in Fig. 16B. As a result, a gap between the specimen treatment chip 20 and the heat generator 41 is eliminated as with the embodiment 1. Thus, temperature control for the specimen treatment chip 20 can be accurately performed. Simply moving the moving member 60 enables the connector 50 to be connected to the connecting port 22 and the elastic member 42 to be pressed on the specimen treatment chip 20. This enables improvement in operability when the specimen treatment chip 20 is mounted, as with the embodiment 1.

As illustrated in Fig. 16A, in the embodiment 2, the connector 50 is disposed so as to be laterally adjacent to the heating unit 40. This allows another member for treating a specimen, such as a magnet, to be easily disposed in a space region below the specimen treatment chip 20 placed in the placement part 30.

The heating unit 40 may be disposed on each of upper and lower sides of the specimen treatment chip 20. That is, the heating unit 40 may be disposed on an opposite side to the connector 50 with respect to the specimen treatment chip 20 placed in the placement part 30, like the embodiment 1, and the heating unit 40 may be provided on the same side as the connector 50 with respect to the specimen treatment chip 20 placed in the placement part 30, like the embodiment 2.

### <Embodiment 3>

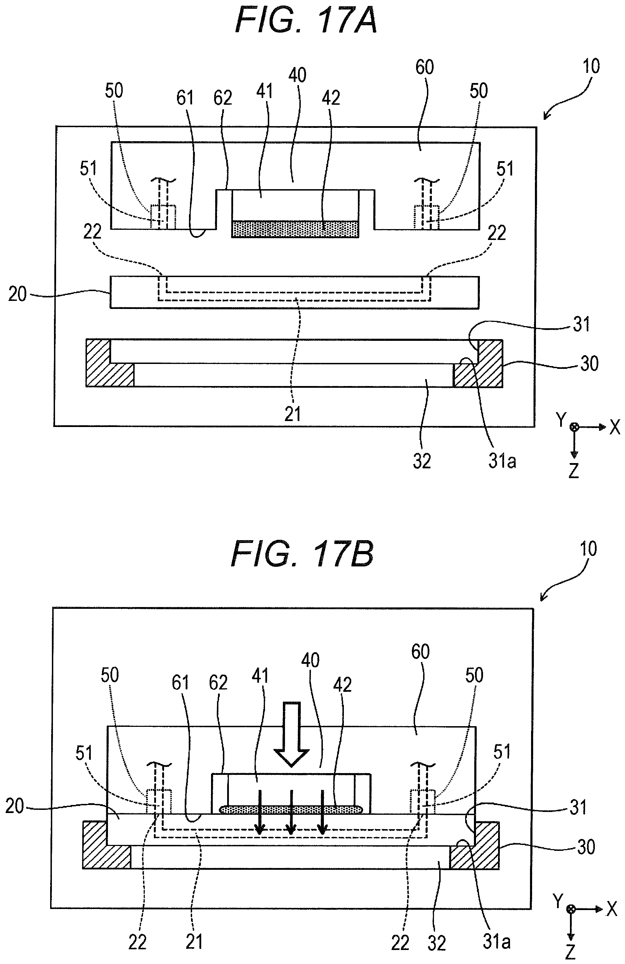

As illustrated in Figs. 17A and 17B, a heating unit 40 is provided in a recessed portion 62 formed in a lower surface 61 of a moving member 60 in an embodiment 3. The connector 50 is formed near an outlet of a hole 51, as a part of a moving member 60. The connector 50 may be formed as a member separated from the moving member 60, like the embodiment 2. The lower surface 61 of the moving member 60 is a bonding surface to the connector 50, or a plane defining a lower surface of the connector 50. Another structure of the embodiment 3 is similar to that of the embodiment 2.

As illustrated in Fig. 17A, the recessed portion 62 is recessed upward from the lower surface 61. The recessed portion 62 has a height that is larger than a thickness of the heat generator 41, and that is smaller than a thickness of the heat generator 41 and the elastic member 42. As a result, when the moving member 60 is moved downward to press the elastic member 42 of the specimen treatment chip 20, the heat generator 41 and the specimen treatment chip 20 are brought into close contact with each other through the elastic member 42, and the connector 50 and the connecting port 22 are connected to each other, as illustrated in Fig. 17B. As illustrated in Fig. 17A, the recessed portion 62 has an inner dimension larger than the elastic member 42 in a horizontal plane. This causes a clearance between the elastic member 42 and a side surface of the recessed portion 62. Thus, the elastic member 42 expanded in the horizontal plane by being pressed is prevented from extending to under the lower surface 61 to obstruct close contact between the connector 50 and the specimen treatment chip 20.

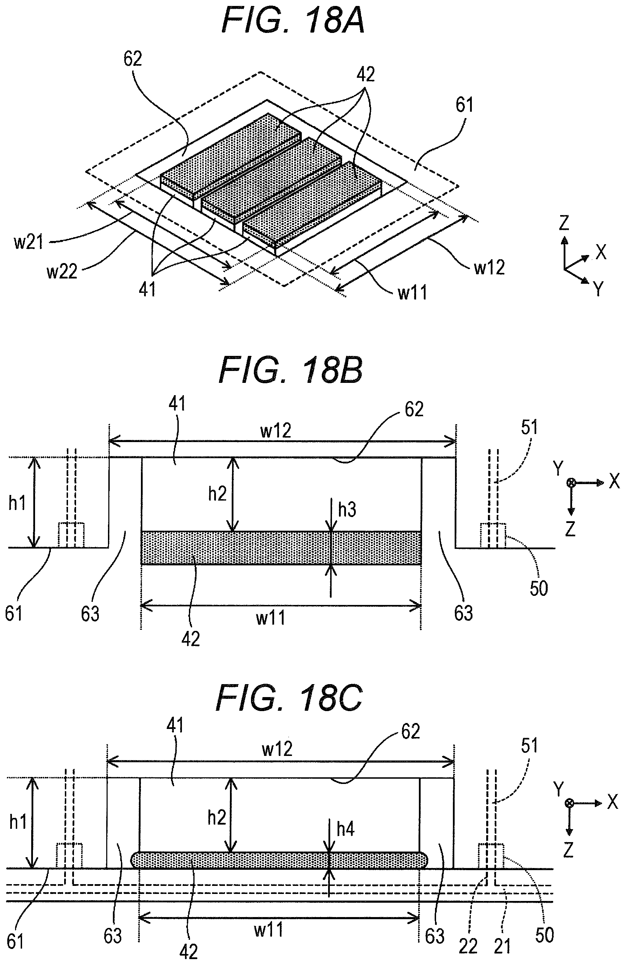

Figs. 18A is an illustration of the vicinity of the elastic member 42 illustrated in Fig. 17A, as viewed from a lower surface 61 side of the moving member 60. As illustrated in Fig. 18A, three pairs of the heat generator 41 and the elastic member 42 are disposed in a Y-axis direction, for example, as with the specific structure of the embodiment 1. In the horizontal plane, the three elastic members 42 are positioned in the recessed portion 62. That is, the recessed portion 62 has an inner dimension in an X-axis direction, larger than an outer dimension of each of the three elastic members 42 in the X-axis direction, and has an inner dimension in a Y-axis direction, larger than an outer dimension of the three elastic members 42 in total in the Y-axis direction. Specifically, as illustrated in Fig. 18A, the outer dimension of each of the three elastic members 42 in the X-axis direction is indicated as w11, and the outer dimension of the three elastic members 42 in total in the Y-axis direction is indicated as w21. Meanwhile, a length of the recessed portion 62 in the X-axis direction is indicated as w12, a length of the recessed portion 62 in the Y-axis direction is indicated as w22. The w12 is more than the w11, and the w22is more than the w21.

Figs. 18B and 18C are respectively enlarged views of the vicinity of the recessed portions 62 of Figs. 17A and 17B. As illustrated in Fig. 18B, a height of the recessed portion 62 is indicated as h1, a thickness of the heat generator 41 is indicated as h2, and a thickness of the elastic member 42 is indicated as h3. The h1 is more than the h2, and is less than a total of the h2 and the h3. This causes the heat generator 41 and the specimen treatment chip 20 to be brought into close contact with each other through the elastic member 42, and causes the connector 50 and the connecting port 22 to be connected to each other, as illustrated in Fig. 18C.

A difference between a height h1 of the recessed portion 62, and a sum of thicknesses h2 + h3 of the heat generator 41 and the elastic member 42, is 1.7 mm or less. A force applied to an upper surface of the specimen treatment chip 20 is a total of a force pressing the elastic member 42 on the specimen treatment chip 20 and a force allowing the connector 50 to be connected to the connecting port 22. Thus, when the difference between the h1 and the "h2 + h3" is set to a small value as described above, a force to be applied to the upper surface of the specimen treatment chip 20 is reduced. As a result, it is possible to prevent deformation of the specimen treatment chip 20 and clogging of the flow channel 21 caused by deformation of the specimen treatment chip 20. When the specimen treatment chip 20 is heated, the specimen treatment chip 20 is particularly liable to be deformed. Thus, setting each of the h1 and the "h2 + h3" to a small value as described above is particularly effective in preventing deformation of the specimen treatment chip 20 and clogging of the flow channel 21.

In the embodiment 3, a difference between the height h1 of the recessed portion 62 and the thickness h2 of the heat generator 41 is 0.3 mm. When the elastic member 42 has a thickness of 0.3 mm or more and 2.0 mm or less, a difference between the height h1 of the recessed portion 62, and the sum of thicknesses h2 + h3 of the heat generator 41 and the elastic member 42, is 0 mm with the elastic member 42 having a minimum thickness of 0.3 mm, and is 1.7 mm with the elastic member 42 having a maximum thickness of 2.0 mm. Thus, in the embodiment 3, a difference between the height h1 of the recessed portion 62, and the sum of thickness h2 + h3 of the heat generator 41 and the elastic member 42, is set to 1.7 mm or less. The difference between the height h1 of the recessed portion 62, and the sum of thicknesses h2 + h3 of the heat generator 41 and the elastic member 42, is not limited to the setting of 1.7 mm or less, and may be set in accordance with a height of the recessed portion 62, a thickness of the heat generator 41, and a thickness of the elastic member 42.

Even in the embodiment 1 illustrated in Figs. 1A and 1B, an upper surface of the heat generator 41 retracts downward from the bottom surface 31a of the placement part 30, and the elastic member 42 projects upward from the bottom surface 31 a. Even in this case, when a thickness of a portion of the elastic member 42, projecting upward from the bottom surface 31a, is set small, a force to be applied to a lower surface of the specimen treatment chip 20 decreases. This case also enables deformation of the specimen treatment chip 20 and clogging of the flow channel 21 to be prevented.

As illustrated in Fig. 18B, the recessed portion 62 has an inner dimension larger than the elastic member 42, so that a clearance 63 is provided between the left end of the elastic member 42 and the left side surface of the recessed portion 62, and between the right end of the elastic member 42 and the right side surface of the recessed portion 62. Likewise, as can be seen with reference to Fig. 18A, a clearance is also provided between the Y-axis positive side end of the elastic member 42 on a Y-axis positive side and the side surface of the recessed portion 62 on the Y-axis positive side, and between the Y-axis negative side end of the elastic member 42 on a Y-axis negative side and the side surface of the recessed portion 62 on the Y-axis negative side. As a result, as illustrated in Fig. 18C, the elastic member 42 expanded in the horizontal plane by being pressed is prevented from extending to under the lower surface 61 to obstruct close contact between the connector 50 and the specimen treatment chip 20.

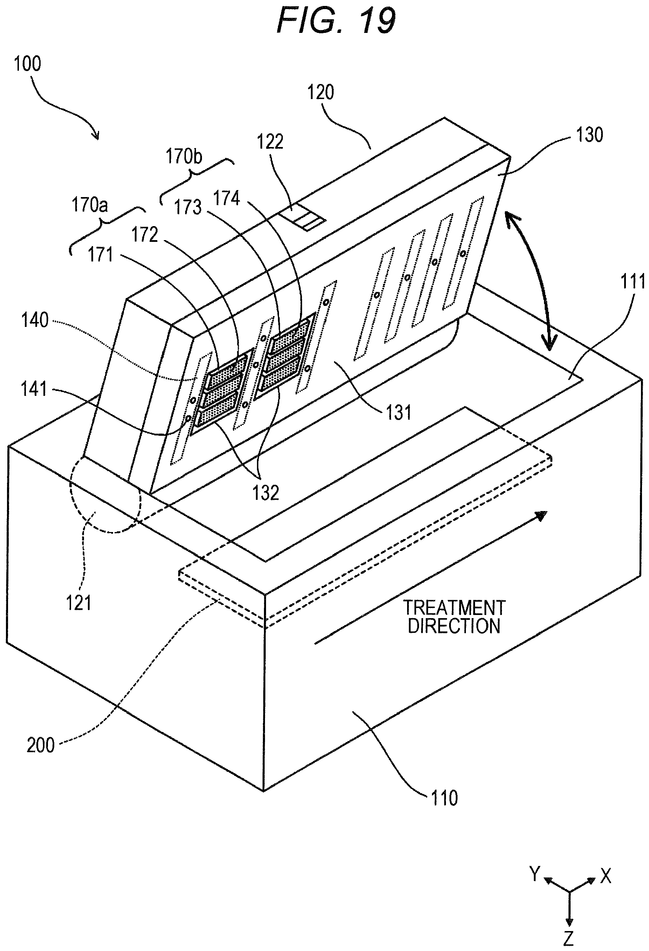

The embodiment 3 is specifically formed as illustrated in Fig. 19.

As illustrated in Fig. 19, two recessed portions 132 are formed in a lower surface 131 of the moving member 130. The recessed portion 132 on the left side is provided with three heating units 170a, and the recessed portion 132 on the right side is provided with three heating units 170b. The connector 140 is formed near an outlet of a hole 141, as a part of the moving member 130. Another specific structure of the embodiment 3 is similar to that of the embodiment 1.

### <Embodiment 4>

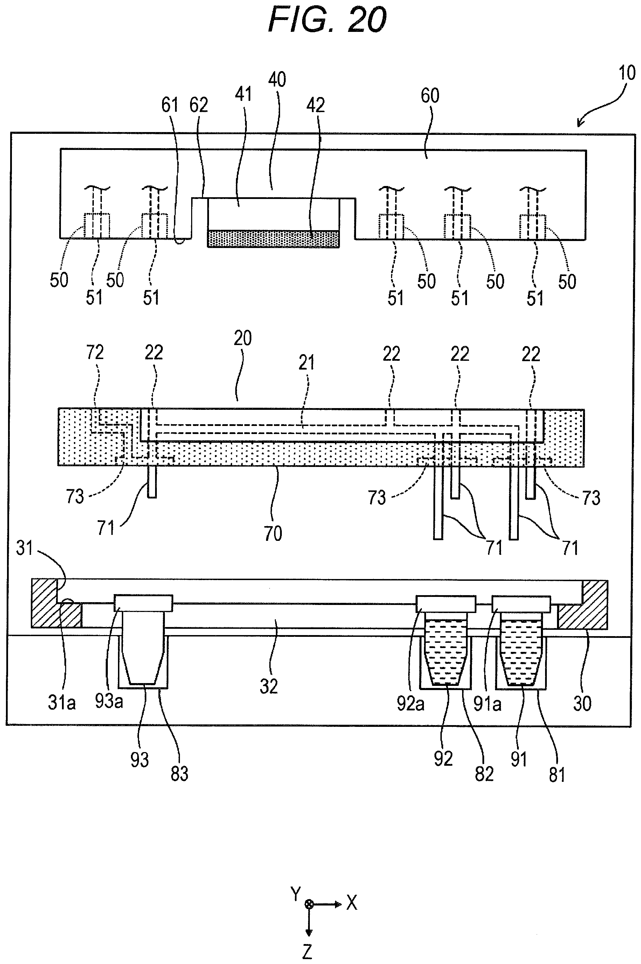

As illustrated in FIG. 20, a recessed portion 62 is formed in a lower surface 61 of a moving member 60, and a heating unit 40 is provided in the recessed portion 62 in an embodiment 4, as with the embodiment 3. The moving member 60 is provided with five connectors 50. Even in this case, the lower surface 61 of the moving member 60 is a plane defining a lower surface of the connector 50, as with the embodiment 3.

The specimen treatment chip 20 is composed of a member including a flow channel 21 and a connecting port 22, and a cartridge 70 provided on a lower surface of the member. The specimen treatment chip 20 includes four connecting ports 22 corresponding to the four connectors 50 on the right side. The cartridge 70 is provided in its lower surface with a plurality of pipes 71, and in its upper surface with one connecting port 72. The connecting port 72 corresponds to the connector 50 at the left end. In a horizontal plane, the cartridge 70 has a size that is substantially identical to that of a recessed portion 31 of a placement part 30.

A specimen treatment apparatus 10 is provided in its inside with a reagent containing section 81, a specimen containing section 82, and a sample containing section 83. The reagent containing section 81 is provided with a reagent container 91 that contains a reagent. The specimen containing section 82 is provided with a specimen container 92 that contains a specimen. The sample containing section 83 is provided with a sample container 93 for containing a sample after treatment is finished. The inside of the reagent container 91 is open upward through its upper end 91 a. The inside of the specimen container 92 is open upward through its upper end 92a. The inside of the sample container 93 is open upward through its upper end 93a. The cartridge 70 is provided in its lower surface with recessed portions 73 into which the corresponding upper ends 91a, 92a, and 93a are fitted. Another structure of the embodiment 4 is similar to that of the embodiment 3.

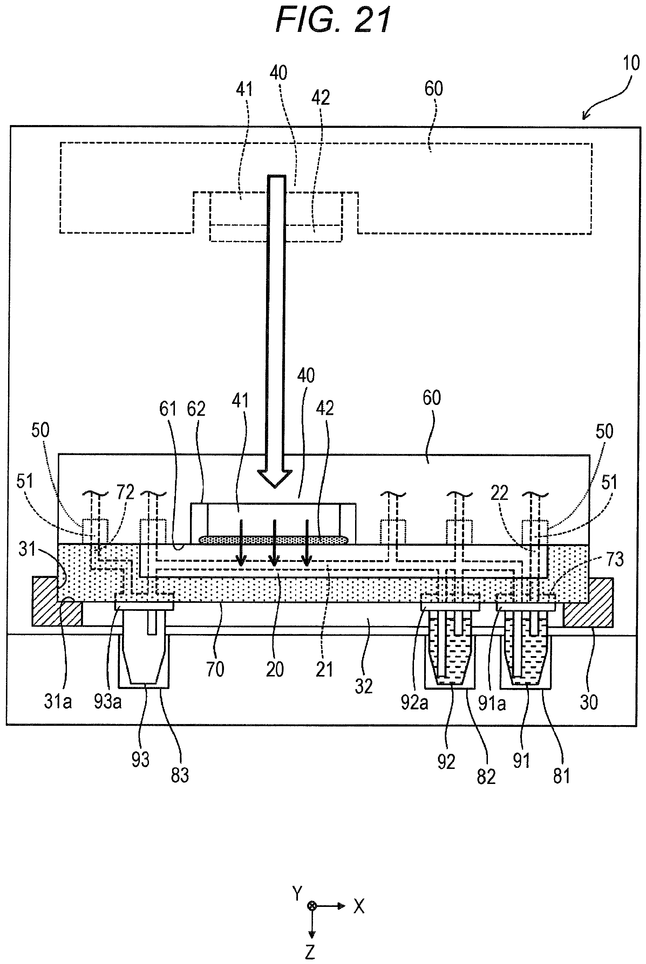

When the moving member 60 is moved downward from a state of Fig. 20, the four connectors 50 on the right side are connected to the corresponding connecting ports 22, and the connector 50 at the left end is connected to the connecting port 72, as illustrated in Fig. 21. Then, an elastic member 42 is pressed on an upper surface of the specimen treatment chip 20. As a result, temperature control for the specimen treatment chip 20 by using the heating unit 40 can be accurately performed even in the embodiment 4. Simply moving the moving member 60 enables the connectors 50 to be connected to the corresponding connecting ports 22 and 72, and the elastic member 42 to be pressed on the specimen treatment chip 20. This enables improvement in operability when the specimen treatment chip 20 is mounted, even in the embodiment 4.

When the moving member 60 is moved downward from a state of Fig. 20, a bottom surface of the cartridge 70 is brought into contact with a bottom surface 31a of the recessed portion 31. As a result, the upper end 91 a of the reagent container 91, the upper end 92a of the specimen container 92, and the upper end 93a of the sample container 93, are fitted into the corresponding three recessed portions 73 formed in the bottom surface of the cartridge 70. At this time, the inside of the reagent container 91 is sealed while two pipes 71 are inserted into the inside of the reagent container 91, the inside of the specimen container 92 is sealed while two pipes 71 are inserted into the inside of the specimen container 92, and the inside of the sample container 93 is sealed while one pipe 71 is inserted into the inside of the sample container 93.

When a specimen is treated, air is injected into each of the two connecting ports 22 from the right end, for example. A reagent is injected into the third connecting port 22 from the right. A waste fluid is extracted from the fourth connecting port 22 from the right. The connecting port 72 is used to drain air. When the five connectors 50 are connected to the corresponding reagent container 91, specimen container 92, and sample container 93 through the corresponding connecting ports 22 and 72, as illustrated in Fig. 21, the specimen and the reagent can be fed to the flow channel 21 positioned below the heating unit 40. Then, a sample heated by the heating unit 40 can be fed to the sample container 93. Thus, treatment of a specimen can be smoothly performed even in the embodiment 4.

### <Embodiment 5>

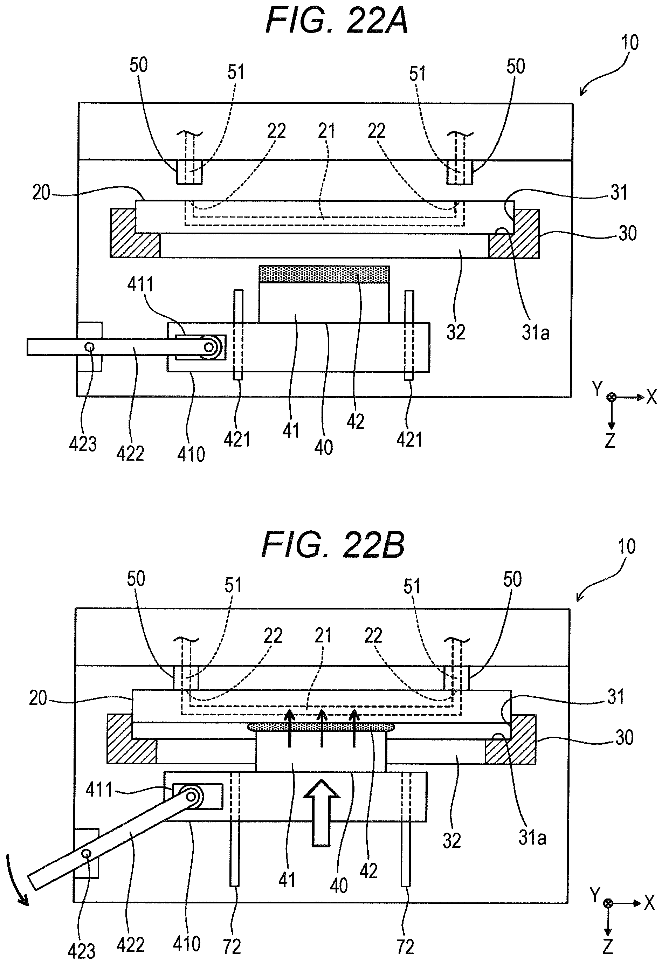

As illustrated in Fig. 22A, a connector 50 is fixed to the inside of a specimen treatment apparatus 10 in an embodiment 5. A specimen treatment chip 20 is placed in a placement part 30 through an opening (not illustrated) or the like. Below the placement part 30, a moving member 410, two rails 421, an operation lever 422, and a shaft member 423, are provided inside the specimen treatment apparatus 10. The heating unit 40 is provided in an upper portion of the moving member 410. Another structure of the embodiment 5 is similar to that of the embodiment 1.

As illustrated in Fig. 22A, the moving member 410 is supported by the rails 421 so as to be vertically movable. The moving member 410 is provided with a hole 41 passing through it in a Y-axis direction. The hole 411 has a shape extending in an X-axis direction as viewed from the Y-axis direction. The operation lever 422 has a right end portion that is fitted into the hole 411 so as to be movable in the X-axis direction inside the hole 411. The operation lever 422 has a left end portion that is positioned outside the specimen treatment apparatus 10. The operation lever 422 is supported by the shaft member 423 so as to be rotatable around the Y-axis.

When an operator presses down the left end portion of the operation lever 422 from a state illustrated in Fig. 22A, the operation lever 422 is turned to cause the right end portion of the operation lever 422 to press up the moving member 410 as illustrated in Fig. 22B. This causes the heating unit 40 to be moved upward to press an elastic member 42 on the specimen treatment chip 20. The specimen treatment chip 20 is pressed up through the elastic member 42, so that the connector 50 is connected to a connecting port 22. As a result, temperature control for the specimen treatment chip 20 by using the heating unit 40 can be accurately performed even in the embodiment 5. Simply operating the operation lever 422 to move the moving member 60 enables the connector 50 to be connected to the connecting port 22 and the elastic member 42 to be pressed on the specimen treatment chip 20. This enables improvement in operability when the specimen treatment chip 20 is mounted, even in the embodiment 5.

While the moving member 60 is pressed up when an operator presses up the operation lever 422, the moving member 60 may be automatically pressed up by using a motor or the like.

### <Embodiment 6>

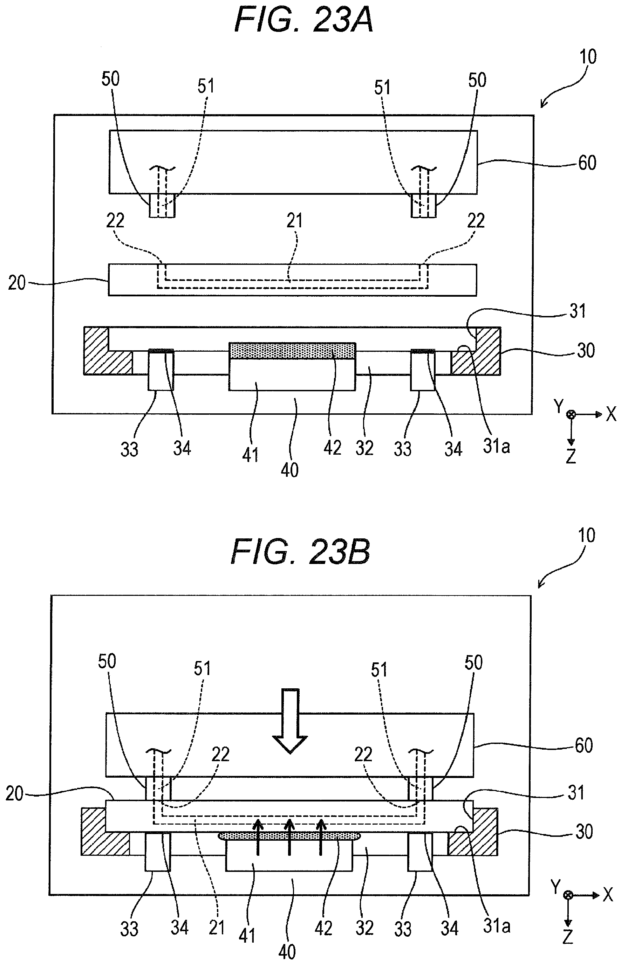

As illustrated in Figs. 23A and 23B, two support members 33 are disposed on an opposite side to the corresponding two connectors 50 with respect to a specimen treatment chip 20 placed in a placement part 30 in an embodiment 6. The two support members 33 are provided in a hole 32 in the placement part 30 inside a specimen treatment apparatus 10. In an XY-plane, a position of a support member 33 is identical to a position of the corresponding connector 50. In the embodiment 6, a cushioning member 34 is disposed on an upper surface of the support member 33. The cushioning member 34 is formed of rubber, for example. Another structure of the embodiment 6 is similar to that of the embodiment 1.

When the moving member 60 is moved downward from a state of Fig. 23A, the connector 50 is connected to a connecting port 22, and an elastic member 42 is pressed on the specimen treatment chip 20, as illustrated in Fig. 23B, as with the embodiment 1. At this time, in the embodiment 6, the support member 33 positioned directly below the connector 50 supports a lower surface of the specimen treatment chip 20. As a result, the connector 50 and the connecting port 22 can be reliably connected to each other. Then, a force applied to the specimen treatment chip 20 from the connector 50 can prevent the specimen treatment chip from bending.

In Fig. 23B, the support member 33 supports the lower surface of the specimen treatment chip 20 through the cushioning member 34. This enables a force to be applied to the specimen treatment chip 20 from the support member 33 to be distributed, so that deformation of the specimen treatment chip 20 can be reduced as compared with a case where the support member 33 directly supports the specimen treatment chip 20. As a result, breakage of the specimen treatment chip 20 can be prevented.

### <Embodiment 7>

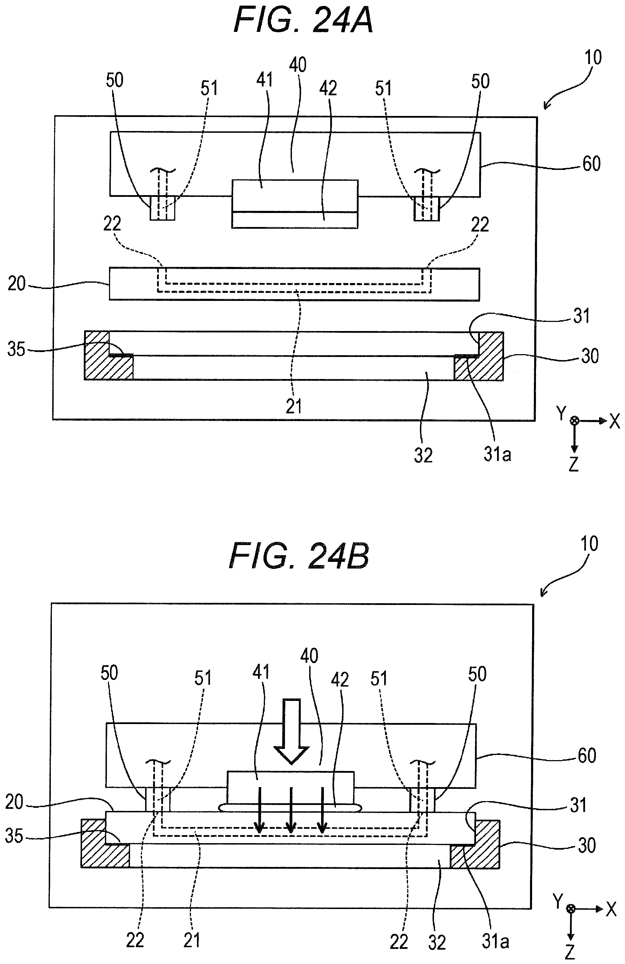

As illustrated in Figs. 24A and 24B, a heating unit 40 of an embodiment 7 is a heater block including a heat generator 41 that generates heat, and an elastic member 42 formed integrally with the heat generator 41, in contrast to the embodiment 2. The elastic member 42 of the embodiment 7 is formed of a material with flexibility and high thermal conductivity. In the embodiment 7, an elastic member 35 is provided on a bottom surface 31a of a placement part 30, in contrast to the embodiment 2. The elastic member 35 is formed of rubber, for example. Another structure of the embodiment 7 is similar to that of the embodiment 2.

When a moving member 60 is moved downward from a state of Fig. 24A, a connector 50 is connected to a connecting port 22, and the elastic member 42 is pressed on a specimen treatment chip 20, as illustrated in Fig. 24B, as with the embodiment 2. At this time, a lower surface of the specimen treatment chip 20 is supported by the bottom surface 31a of the placement part 30 through the elastic member 35 in the embodiment 7. The bottom surface 31 a is a supporting surface that supports the specimen treatment chip 20. This enables a force to be applied to the specimen treatment chip 20 from the bottom surface 31a to be distributed, so that deformation of the specimen treatment chip 20 can be reduced as compared with a case where the bottom surface 31 a directly supports the specimen treatment chip 20. As a result, breakage of the specimen treatment chip 20 can be prevented.

### <Embodiment 8>

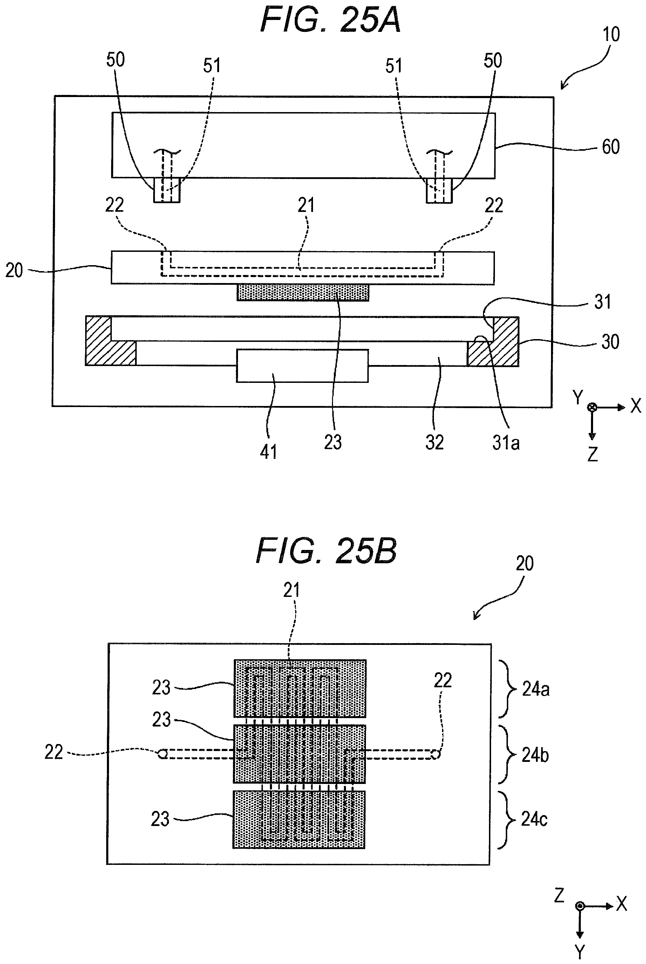

As illustrated in Fig. 25A, an elastic member is provided in an upper surface of a heat generator 41 instead of a lower surface of a specimen treatment chip 20 in an embodiment 8. That is, the specimen treatment chip 20 of the embodiment 8 is composed of a member including a flow channel 21 and a connecting port 22, and an elastic member 23 provided on a lower surface of the member. The elastic member 23 is bonded to the lower surface of the specimen treatment chip 20 with elastic silicone rubber-based adhesive, for example.

As illustrated in Fig. 25B, the flow channel 21 of the specimen treatment chip 20 of the embodiment 8 meanders with a predetermined amplitude. On the lower surface of the specimen treatment chip 20, the elastic members 23 are individually fixed to a first region 24a on a Y-axis negative side across the middle portion of the meander, a second region 24b at the middle portion of the meander, and a third region 24c on a Y-axis positive side across the middle portion of the meander. Three elastic members 23 aligning in the Y-axis direction, corresponding to three respective heat generators 41, are provided inside a specimen treatment apparatus 10. Another structure of the embodiment 8 is similar to that of the embodiment 1.

When a moving member 60 is moved downward from a state of Fig. 25A, a connector 50 is connected to the connecting port 22, and the elastic member 23 is pressed on the heat generator 41, as with the state illustrated in Fig. 1B. As a result, temperature control for the specimen treatment chip 20 by using the heat generator 41 can be accurately performed even in the embodiment 8. Simply moving the moving member 60 enables the connector 50 to be connected to the connecting port 22 and the elastic member 23 to be pressed on the heat generator 41. This enables improvement in operability when the specimen treatment chip 20 is mounted, even in the embodiment 8.

The first region 24a, the second region 24b, the third region 24c of the specimen treatment chip 20 are heated by respective different heat generators 41 through respective elastic members 23. As a result, three temperatures can be applied to a sample by only allowing the sample to flow through the flow channel 21 meandering, so that thermal cycle treatment during the nucleic acid amplification can be easily performed, for example.

According to the embodiment 8, the elastic member 23 is provided on the lower surface of the specimen treatment chip 20, so that no elastic member is needed to be provided on the heat generator 41. The elastic member 23 is provided on the lower surface of the specimen treatment chip 20, so that a fresh elastic member 23 is to be used every time a fresh specimen treatment chip 20 is placed. When a fresh elastic member is used every time as described above, a deteriorated elastic member can be prevented from being used. As a result, temperature control by using the heat generator 41 can be more reliably performed. Meanwhile, when the elastic member 42 is provided on the heat generator 41 like the embodiments 1 to 7, no elastic member 23 is needed to be provided on the specimen treatment chip 20. As a result, costs for the specimen treatment chip 20 can be reduced.

When the elastic member 23 is provided on the specimen treatment chip 20 like the embodiment 8, protector paper is preliminarily stuck on the lower surface of the elastic member 23. This enables the elastic member 23 to be protected during a period from manufacturing of the specimen treatment chip 20 to usage thereof through distribution.

When the heat generator 41 is provided above the specimen treatment chip 20 placed in the placement part 30 like the embodiments 2 to 4, the elastic member 23 is provided on the upper surface of the specimen treatment chip 20. When the heat generator 41 is provided above as well as below the specimen treatment chip 20 placed in the placement part 30, the elastic member 23 is provided on each of the upper surface and the lower surface of the specimen treatment chip 20.

## Claims

1. A specimen treatment apparatus that treats a specimen by using a specimen treatment chip including a flow channel and a connecting port communicating with the flow channel, the specimen treatment apparatus comprising:
a placement part in which the specimen treatment chip is placed;
a connector provided to be engaged with the connecting port to allow a sample containing a specimen to be injected into the flow channel through the connector; and
a heating unit that is pressed on the specimen treatment chip and heats the specimen treatment chip while the connector is connected to the connecting port of the specimen treatment chip placed in the placement part.

2. The specimen treatment apparatus according to claim 1, wherein the heating unit includes a heat generator that generates heat and an elastic member provided on the heat generator.

3. The specimen treatment apparatus according to claim 1, wherein the heating unit includes a heat generator that generates heat and an elastic member that is formed integrally with the heat generator.

4. The specimen treatment apparatus according to any one of claims 1 to 3, further comprising a moving member that can connect the connector to the connecting port and can press the heating unit on the specimen treatment chip.

5. The specimen treatment apparatus according to claim 4, wherein the moving member moves the connector to connect the connector to the connecting port, and to press the heating unit on the specimen treatment chip.

6. The specimen treatment apparatus according to any one of claims 1 to 3, further comprising: a body; and a lid part that is supported by the body to be openable and closable, wherein
the lid part is configured so as to be closed to connect the connector to the connecting port, and to press the heating unit on the specimen treatment chip.

7. The specimen treatment apparatus according to claim 6, wherein the connector is provided in the lid part.

8. The specimen treatment apparatus according to any one of claims 1 to 7, wherein
the specimen treatment chip includes a plurality of connecting ports, and
a plurality of connectors is provided to be engaged with the plurality of connecting ports, respectively, to inject a reagent for treating a specimen into the flow channel through the plurality of connecting ports.

9. The specimen treatment apparatus according to claim 8, wherein the heating unit is disposed on an opposite side to the plurality of connectors with respect to the specimen treatment chip placed in the placement part.

10. The specimen treatment apparatus according to claim 2 or 3, further comprising a locking part that locks a member for connecting the connector to the connecting port and pressing the elastic member on the specimen treatment chip.

11. The specimen treatment apparatus according to claim 2 or 3, wherein
the heating unit and the connector are provided in the member for connecting the connector to the connecting port and pressing the elastic member on the specimen treatment chip,
the heat generator is disposed on the elastic member, and
the connector is disposed to be lateral to the heating unit.

12. The specimen treatment apparatus according to any one of claims 1 to 11, wherein pressure to be applied to the specimen treatment chip is 85.6 kPa or more and 839.5 kPa or less while the connector is connected to the connecting port.

13. A specimen treatment method for treating a specimen by using a specimen treatment chip that includes a flow channel and a connecting port communicating with the flow channel, the specimen treatment method comprising the steps of:
connecting a connector to the connecting port, the connector allowing a sample containing a specimen to be injected into the flow channel through the connector; and
pressing a heating unit that heats the specimen treatment chip, on the specimen treatment chip.

14. A specimen treatment method for treating a specimen by using a specimen treatment chip including a flow channel and a connecting port communicating with the flow channel, the specimen treatment method comprising the step of
heating the specimen treatment chip by a heating unit while a connector is connected to the connecting port and the heating unit is pressed on the specimen treatment chip.

15. A specimen treatment chip, comprising:
a flow channel;
a connecting port communicating with the flow channel; and
an elastic member fixed to a region corresponding to a portion of the flow channel where temperature control is needed.
